# EUROPEAN PATENT SPECIFICATION

(11) **EP 2 037 821 B1**
(45) Date of publication and mention of the grant of the patent: **22.06.2022**
(21) Application number: 07812507.7
(22) Date of filing: 29.06.2007
(51) Int. Cl.: A61B 17/3207, A61B 17/00, A61M 25/01, A61B 17/22

(54) **ATHERECTOMY DEVICES**
ATHEREKTOMIEVORRICHTUNGEN
DISPOSITIFS D'ATHÉRECTOMIE

(30) Priority: 30.06.2006 US 806417 P; 26.07.2006 US 820475 P; 19.10.2006 US 551191; 06.12.2006 US 567715
(43) Date of publication of application: 25.03.2009
(60) Divisional application: 17198488.3
(73) Proprietor: Atheromed, Inc., Menlo Park, CA 94025 (US)
(72) Inventor: TO, John, Newark, CA 94560 (US); DANEK, Christopher James, San Carlos, CA 94070 (US); ESCUDERO, Paul, Redwood City, CA 94061 (US)
(74) Representative: Philips Intellectual Property & Standards
(86) International application number: PCT/US2007/072570
(87) International publication number: WO 2008/005888

(56) References cited:
- WO-A1-00/54659
- WO-A1-92/14506
- WO-A1-2005/123169
- WO-A2-01/64115
- US-A- 5 728 129
- US-A- 5 910 150
- US-A- 6 027 450
- US-A- 6 156 046

## Description

### CROSS-REFERENCE

This application is a continuation in part of U.S. Application No. 11/567,715, entitled "Atherectomy Devices and Methods" filed December 6, 2006, which is a continuation of U.S. Application No. 11/551,191 entitled "Atherectomy Devices and Methods" filed October 19, 2006, which is a non-provisional of U.S. Provisional Application Serial No. 60/806,417 entitled "Atherectomy Device" filed June 30, 2006 and is a non-provisional of U.S. Provisional Application Serial No. 60/820,475 entitled "Atherectomy Device" filed July 26, 2006.

### BACKGROUND OF THE INVENTION

### Field of the Invention

The devices and exemplary methods described below generally relate to treatment of occluded body lumens. In particular, the present devices and exemplary method relate to removal of the occluding material from the blood vessels as well as other body lumens.

### Description of the Background Art

Atherosclerosis is a progressive disease. In this disease, lesions of the arteries are formed by accumulation of plaque and neointimal hyperplasia causing an obstruction of blood flow. Often plaque is friable and may dislodge naturally or during an endovascular procedure, leading to embolization of a downstream vessel.

Endovascular clearing procedures to reduce or remove the obstructions to restore luminal diameter allows for increased blood flow to normal levels are well known. Removing the plaque has the effect of removing diseased tissue and helps to reverse the disease. Maintaining luminal diameter for a period of time (several to many weeks) allows remodeling of the vessel from the previous pathological state to a more normal state. Finally, it is the goal of an endovascular therapy to prevent short term complications such as embolization or perforation of the vessel and long term complications such as ischemia from thrombosis or restenosis.

Various treatment modalities may help to accomplish treatment goals. In atherectomy, plaque is cut away, or excised. Various configurations are used including a rotating cylindrical shaver or a fluted cutter. The devices may include shielding by a housing for safety. The devices may also remove debris via trapping the debris in the catheter, in a downstream filter, or aspirating the debris. In some cases a burr may be used instead of a cutter, particularly to grind heavily calcified lesions into very small particle sizes. Aspiration may also be used with a burr-type atherectomy device.

Balloon angioplasty is another type of endovascular procedure. Balloon angioplasty expands and opens the artery by both displacing the plaque and compressing it. Balloon angioplasty is known to cause barotrauma to the vessel from the high pressures required to compress the plaque. This trauma leads to an unacceptably high rate of restenosis. Furthermore, this procedure may not be efficient for treatment of elastic-type plaque tissue, where such tissue can spring back to occlude the lumen.

When clearing such obstructions it is desirable to protect the vessel wall or wall of the body lumen being cleared and to debulk substantially all of a lesion. In additional cases, the procedure that clears obstructions may also be coupled with placement of an implant within the lumen. For example, it may be desirable to deploy a stent to maintain patency of a vessel for a period of time and/or to achieve local drug delivery by having the stent elute a drug or other bioactive substance.

On their own, stents fail to perform well in the peripheral vasculature for a variety of reasons. A stent with the necessary structural integrity to supply sufficient radial force to reopen the artery often does not perform well in the harsh mechanical environment of the peripheral vasculature. For example, the peripheral vasculature encounters a significant amount of compression, torsion, extension, and bending. Such an environment may lead to stent failure (strut cracking, stent crushing, etc.) that eventually compromises the ability of the stent to maintain lumen diameter over the long-term. On the other hand, a stent that is able to withstand the harsh mechanical aspects of the periphery often will not supply enough radial force to open the vessel satisfactorily. In many cases, medical practitioners desire the ability to combine endovascular clearing procedures with stenting. Such stenting may occur prior to, after, or both before and after the endovascular clearing procedure.

WO 92/14506 relates to a device which comprises a steerable elongated tube or cannula (22), a resilient member (24) to deflect the cannula, and a rectifying element (26) preventing the elastic member (24) from influencing the cannula. The rectifying element (26) and the spring element (24) are capable of relative axial movement, so that the straightening member (26) can be positioned to allow or prohibit the elastic element bend the cannula.

US 5728129 discloses a distal atherectomy catheter for removing obstructions, plaque, stenosis, occlusions, or the like from an artery or coronary vessel. The catheter comprises a flexible, hollow catheter tube. A cutting element is located within a cylindrical housing mounted at the distal end of the catheter tube. The cutting element is connected to a hollow, flexible drive shaft concentrically located within the catheter tube. The cutting element housing includes a side opening window or port providing access to the interior of the housing.

US 6027450 describes a method and system for recanalizing a totally or near totally occluded body lumen such as an artery which has an elongated catheter shaft with means such as a cutting or ablating element at the distal end of the shaft to remove occluding material.

WO 2005/123169 relates to a vascular steerage access device having an elongate body and a steerable portion. The access sheath has an outside diameter sufficiently small so that it may be inserted into a vessel and a sufficient length to extend through a patient's circulatory system.

WO 01/64115 discloses a system for ablating material in vein grafts includes an ablation burr that is rotated by a driveshaft.

US 5910150 describes apparatus and methods for performing surgery within a hollow-body organ. A catheter is provided having a longitudinal axis and an end region carrying an end effector, the end region movable to a series of positions along the longitudinal axis and with a selectable orientation relative to the longitudinal axis.

US 6156046 relates to a system having a catheter, a torque member extending longitudinally through a lumen of the catheter, a removal mechanism secured to a distal end of the torque member, and a guidewire having a guide section that extends through a lumen of the removal mechanism. WO 00/54659 discloses a device for removing material having a housing with apertures and a rotating shearing body within.

Accordingly, a need remains for devices that allow for improved atherectomy devices that clear materials from body lumens (such as blood vessels) where the device includes features to allow for a safe, efficient and controlled fashion of shaving or grinding material within the body lumen.

### SUMMARY OF THE INVENTION

According to the invention, there is provided a device for removing material from body lumens as defined in the appended claim 1. Further embodiments of the invention are defined by the dependent claims. Methods do not form part of the claimed inventions.

Devices and exemplary methods described herein provide improved means of clearing obstructions within body lumens, especially the vasculature. The features of the devices and methods allow for controlled removal of occlusive materials. In some variations, the methods and devices also have features to convey the materials away from the operative site without the need to remove the devices from the body lumen. Additional aspects include controlled rates of tissue removal as well as other safety features to prevent accidental cutting of the lumen wall. Although the devices and methods described herein discuss removal of materials from a blood vessel, in certain cases the devices and methods have applicability in other body lumens as well. It should be noted that the variations and features of the devices described below may be incorporated selectively or in combination with a basic device configuration that includes a flexible body having a cutter, where the cutter includes a housing and a cutter, where the housing and cutter are able to rotate relative to each other. Variations include a cutter that rotates within the housing, a housing that rotates about the cutter, and combinations thereof.

One variation of the device described herein includes a device configured to remove material from body structures. The device may be a vascular device and have the required structure and configuration to navigate tortuous anatomy. Alternatively, the device may be a cutter that has features that are desired when used in other parts of the anatomy.

In any case, such a device may include a catheter body having a proximal end and a distal end, a cutter assembly located at the distal end of the catheter body, the cutter assembly comprising a housing having at least one opening and a cutter having at least one cutting surface configured to rotate relative to the housing, where movement of the cutting surface relative to the vessel removes occlusive material, a rotating shaft extending through the catheter body and coupled to the cutter, the shaft having a proximal end adapted to couple to a first rotating mechanism, and a deflecting member extending along the catheter body, such that the deflection member can cause deflection of the cutter assembly relative to an axis of the catheter.

Devices of the present disclosure can also include multiple cutting surfaces. For example, the multiple cutting surfaces may cut tangential to a rotational direction of a cutting head, in a forward direction as the cutting assembly moves distally, and/or in a rearward direction as the cutting assembly is withdrawn proximally. The multiple cutting surfaces can be located on a single cutting head, or may be located on a housing of the cutting assembly. In certain variations of the device, a housing of the cutting assembly may be fully open at a distal end to expose a cutting head. Such a design can incorporate additional safety features to prevent excessive damage to vessel walls.

Variations of the deflecting member may include steerable sheaths adapted to deflect in shape. The steerable sheath may be located internally to a catheter body of the device. Accordingly, the catheter body remains stationary while the sheath can rotate to move a cutting head in an arc about the target body passage.

In some variations the steerable sheath may include a deflecting wire extending through a portion of the sheath, such that axial movement of the deflecting wire deflects the sheath. The deflecting wire can be affixed to the cutter assembly, to a portion of the catheter body that extends out of the deflecting sheath, or to other parts of the device as needed.

The deflecting member can also include a pre-shaped mandrel, or tube where such features are slidable within or relative to the device to produce movement of the cutting head relative to an axis of the device. The devices described herein may have any number of features that allow for locking the device after it is articulated. This feature provides a consistent diameter when sweeping or navigating through the anatomy.

As discussed herein, some variations of the devices have the ability to articulate. This articulation allows for steering the device to the target site as well as creating a sweeping motion of tissue removal. Accordingly, a deflectable sheath used in the device can be rotatable about the catheter body, or about an axis of the catheter.

The devices described herein may have a cutter assembly having a portion of its housing having a curved surface and where the opening forms a plane across the curved surface such that as the cutting surface rotates across the opening, a portion of the cutting surface extends out of the housing through the opening. The cutter assembly may also have various other features as described below that improve the safety of the device as it is articulated while cutting. Furthermore the cutter may have a number of features to impel or drive cut tissue into the cutter assembly for eventual removal by one or more conveying members.

As noted, the devices described herein may have one or more conveying members that convey materials and/or fluids through the device. Such a feature is useful to remove cut tissue and debris from the site during the procedure. In some variations, the device may include multiple conveyors to deliver fluids and remove debris. However, the devices of the present invention may also have containers for use in capturing debris or other materials generated during the procedure.

Another feature for use with the disclosures herein is the use of a grinding burr rotatably coupled to a tip of the device. The burr can be useful to remove tissue that is otherwise not conducive to cutting with the cutter assembly.

In another variation, the disclosure may comprise a device having a straightening tube, with a straight distal portion, a catheter body having a proximal end and a distal end, the catheter body having a flexible section located towards the distal end, such that when located in the straight distal portion of the straightening tube the flexible section is less curved, a cutter assembly located at the distal end of the catheter body, the cutter assembly comprising a housing having at least one opening and a cutter having at least one cutting surface configured to rotate relative to the housing, where movement of the cutting surface removes material, and a rotating shaft extending through the catheter body and coupled to the cutter, the torque shaft having a proximal end adapted to couple to a first rotating mechanism.

In such a case, placement of the straight distal portion over the catheter allows for manipulation of the degree of curvature of the catheter. This feature allows for steering of the device.

As described herein, such a device may have the ability to sweep over an arc to deliver a larger cutting diameter than the diameter of the cutter assembly.

The devices described herein may use a guidewire for advancement through the body. In such cases the devices will have guide-wire lumens located within or about the catheter. Alternatively, a guide-wire section may be affixed to a portion of the device.

Devices of the present disclosure typically include a torque shaft to deliver rotational movement to components in the cutter assembly. Alternatively, a torque shaft or other such assembly may be used to produce the sweeping action described herein. In any case, the torque shaft may include one or more lumens. Alternatively, the torque shaft may be a solid or hollow member. Variations of the torque shaft also include those aspects known in catheter-type devices such as counter-wound coils, stiffening members, etc. In some variations, the torque shaft may have the conveying member integrally formed about the exterior or an interior surface of the shaft. Alternatively, or in combination, the conveying member may be placed on (or within) the torque shaft as described herein.

The disclosure also includes various exemplary not claimed methods of debulking material within body structures. These structures include occluded blood vessels (whether partially or totally occluded), various organs, cavities within the body, or other body lumens.

In one variation an exemplary method includes inserting a catheter body having a cutter assembly within the blood vessel, rotating the cutter assembly to remove the material and form a first opening in the body lumen, deflecting the first cutter assembly relative to an axis of the catheter body, rotating the deflected catheter tip while rotating the cutter assembly to form a second opening in the body lumen where the second is larger than the first opening.

The exemplary methods may include the use of any of the devices or features of the devices described herein. In one variation, the exemplary methods include circulating fluid for contrast to better visualize the obstruction.

As noted herein, combinations of aspects of the devices, systems, and exemplary methods described herein may be combined as needed. Furthermore, combinations of the devices, systems and exemplary methods themselves are within the scope of the disclosure.

### BRIEF DESCRIPTION OF THE DRAWINGS

Fig. 1A illustrates an exemplary variation of a device according to the present disclosure;
Fig. 1B shows an exploded view of the device of Fig. 1A;
Fig. 1C shows a cross sectional view of the cutting assembly;
Figs. 2A shows alignment of the cutting edges with openings of a housing;
Fig. 2B shows a side view of the cutting assembly demonstrating the secant effect;
Fig. 2C illustrates a positive rake angle;
Figs. 3A-3B show a variation of a shielded cutter having a plurality of front cutting surfaces, rear cutting surfaces, and fluted cutting surfaces;
Figs. 4A-4B show another shielded cutter having a plurality of front cutting surfaces and fluted cutting surfaces;
Figs. 5A-5D show a cutter assembly having an open ended housing;
Fig. 5E shows a cutter assembly with the open ended housing removing material from a lumen wall;
Fig. 6 shows a partial cross sectional view of a variation of a torque shaft having counter wound coils;
Fig. 7A shows a variation of a device configured for rapid exchange;
Fig. 7B illustrates an example of centering a tip of a cutting assembly over a guide wire;
Fig. 8A shows a conveyor within the device;
Fig. 8B shows a second conveyor within a torque shaft;
Fig. 9A illustrates articulation of a tip of the device;
Fig. 9B-9D shows sweeping of the cutting assembly;
Fig. 9E illustrates another variation where the catheter body includes a set curve in an area that is adjacent to the cutting assembly;
Fig. 10A shows placement of housing windows to prevent damage to the vessel walls;
Figs. 10B-10C shows placement of features of the cutter assembly that prevent damage to the vessel walls;
Figs. 11A-11E show variations of the device for articulating the cutting assembly;
Figs. 12A-12B show a control system for rotating and articulating the cutter assembly;
Fig. 12C shows a perfusion port at a distal portion of the device;
Fig. 12D shows a cross sectional view of a portion of the catheter hub mechanism that removes debris from the device;
Figs. 13A-13F show additional variations of the device for articulating the cutting assembly;
Fig. 14 shows a device with a burr tip, in accordance with the present claimed invention;
Figs. 15A-15C provide examples of fluid delivery systems;
Fig. 16 shows the device placed within a stent or coil;
Figs. 17A-17B show variations of devices for removing tissue from body lumens;
Figs. 18A-18F show additional variations for centering devices within a lumen;;
Fig. 18G shows a balloon actuated device for treating occlusions;
Figs. 19A-19C show a system for visualizing and crossing total occlusions;
Figs. 20A-20C shows devices described above for visualizing and crossing total occlusions;
Figs. 21A-21B shows a variation of crossing a total occlusion by advancing through layers of a vessel;
Figs. 22A-22F show anchoring means on a guidewire for stabilizing devices of the present disclosure;

### DESCRIPTION OF AN EMBODIMENT

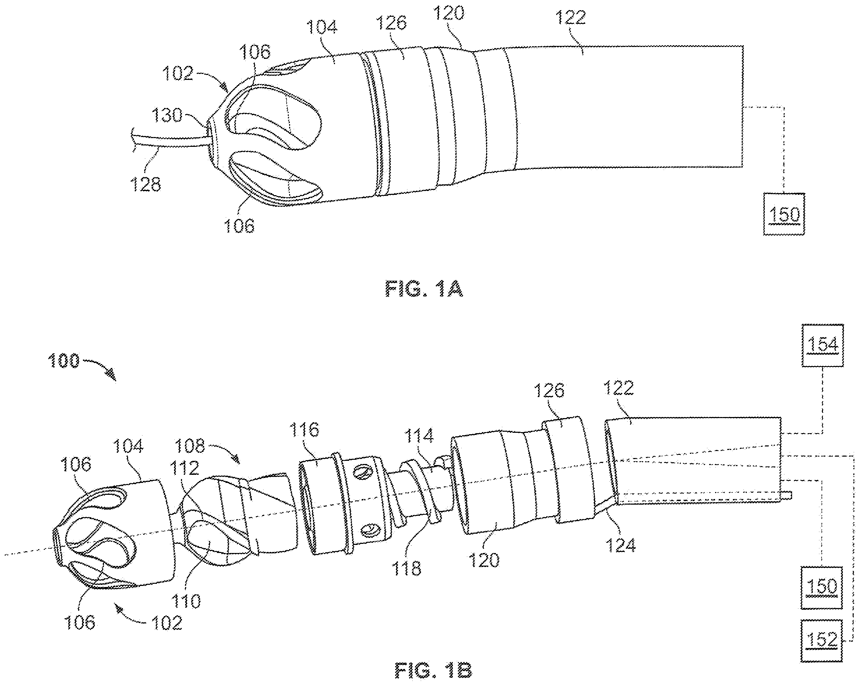

Fig. 1A illustrates an exemplary variation of a device **100** according to the present disclosure. As shown the device **100** includes a cutter assembly **102** affixed to a catheter or catheter body **120.** As shown, the catheter body may be optionally located within an outer sheath **122.** It is noted that the cutter assembly shown in the figures exemplary purposes only. Any cutter assembly may be combined with the other aspects the system The variations of cutter assemblies discussed herein, can be combined with other aspects of the system.

Fig. 1B illustrates an exploded view of the device **100** of Fig. 1A. As shown, in this variation the cutter assembly **102** includes a housing **104** with a plurality of openings **106.** However, additional cutter assembly configurations (as noted below) are combinable with the various aspects of the device configurations discussed herein.

In the illustrated variation, a cutter **108** is located within the housing **104.** This cutter **108** includes one or more flutes **110** each of which includes an edge or cutting surface **112.** The cutter is coupled to a rotating mechanism **150.** In this variation the rotating mechanism couples to the cutter via a torque shaft **114** that transmits rotational energy from the rotating mechanism **150** (e.g., an electric, pneumatic, fluid, gas, or other motor) to the cutter **108.** Variations of the devices include use of a rotating mechanism **150** located entirely within the body of the device **100.** In one variation, the rotating mechanism **150** may be outside of the surgical field (i.e., in a non-sterile zone) while a portion of the device (e.g., the torque shaft - not shown) extends outside of the surgical field and couples to the rotating mechanism. Figure 1B also shows a variation of the device **100** as having a deflecting member **124** where the deflecting member may be a tendon, pull wire, tube, mandrel, a tube or similar structure that causes a distal end of the catheter body to deflect (hereafter referred to as a "sweep sheath") or other such structure. As described in detail below, the devices **100** can have deflecting members to articulate the cutting head and allow for a sweeping motion of cutting.

In another variation, the device **100** may have a catheter body that comprises a soft or flexible portion. In one variation, this soft or flexible portion may be on a single side of the device **100** to allow flexure of the device **100** to articulate the cutting head. The flexure may be obtained with a curved sheath, mandrel, or other means as known to those skilled in the art. In the illustrated variation, the deflecting member **124** comprises a sweep sheath. The sweep sheath **124** has a curved or shaped distal portion, where the curve may be planar or the shaped portion may be a non-planar shape). The distal portion of the sweep sheath is more flexible than a proximal portion of the catheter body. As a result, when the sweep sheath assumes a somewhat straightened shape when in the proximal portion of the catheter body. However, the distal portion of the catheter body is more flexible than the sweep sheath. Accordingly, once the sweep sheath is advanced into the distal portion of the catheter, the catheter assumes the shape or profile of the sweep sheath. This is a way to deflect the cutter assembly off the axis of the catheter. Rotation of the sweep sheath causes the movement of the cutter assembly to sweep in an arc and create an opening larger than a diameter of the catheter itself.

The device **100** may also include a vacuum source or pump **152** to assist in evacuation of debris created by operation of the device. Any number of pumps or vacuum sources may be used in combination with the device. For example, a peristaltic pump may be used to drive materials from the device and into a waste container. Fig. 1B also shows the device **100** coupled to a fluid source **154.** As with the rotating mechanism, the vacuum source and/or fluid source may be coupled to the device from outside the surgical field.

It may be advantageous to rotatably couple the torque shaft to the drive unit electromagnetically, without physical contact. For example, the torque shaft **114** can have magnetic poles installed at the proximal end, within a tubular structure that is attached to the sheath around the torque shaft. The stationary portion of the motor can be built into a handle that surrounds the tubular structure. This allows the continuous aspiration through the sheath without the use of high speed rotating seals.

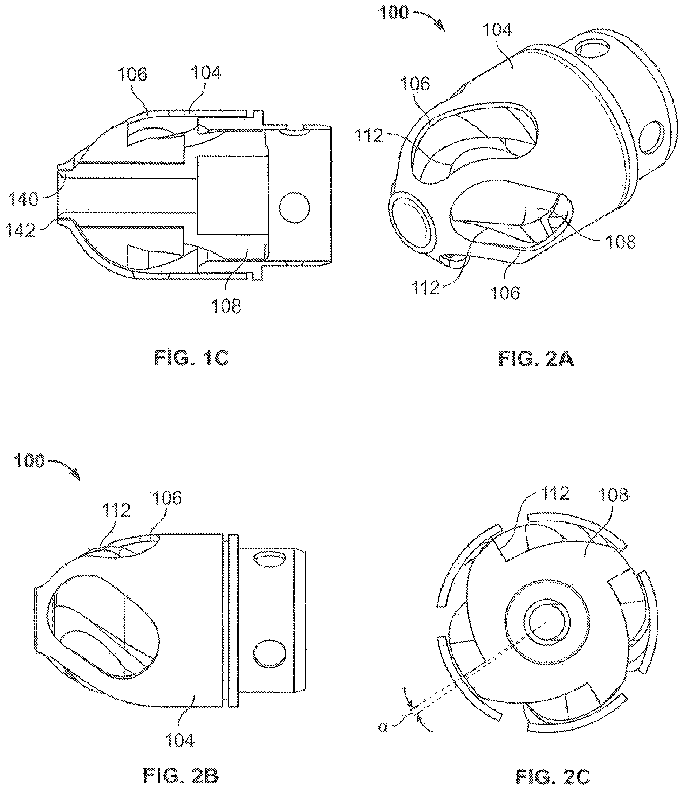

As shown in Fig. 1C, in certain variations, the housing **104** can have a distal nose with a center lumen **142** for receiving a mating piece **140** of the cutter **108.** Such features assist in centering the cutter **104** concentrically inside the housing **104.** As noted below, variations of the devices include the addition of a burr element (as shown below) for grinding hard tissue such as calcified plaque.

The geometry of the cutter **108** and housing **104** can be used to tailor the desired degree of cutting. The housing **104** and orientation of the openings **106** can be used to limit the depth of cutting by the cutter **108.** In addition, the distal end of the housing **104** may be domed shaped while the proximal end may have a cylindrical or other shape. For example, by creating larger windows **106** in the housing a larger portion of cutter **108** may be exposed and the rate of cutting increased (for a given rotation speed). By placing the cutting window **106** on a convex portion of the housing, the debulking effectiveness is much less sensitive to the alignment of the cutter housing to the lesion, than if the window were on the cylindrical portion of the housing. This is a key performance limitation of traditional directional atherectomy catheters. In addition, placement of the window on the convex portion of the housing creates a secant effect (as described below).

Fig. 2A illustrates an additional variation of the device **100** where the openings **106** may be helical slots that may or may not be aligned with the cutting surfaces **112** of the cutter **108.** For aggressive cutting, the slots **106** and cutting edges **112** are aligned to maximize exposure of the tissue to cutting edges. In other words, the cutting edges **112** and openings **106** are in alignment so all cutting edges **112** are exposed at the same time to allow simultaneous cutting. Alternatively, alignment of the openings and edges **112** may be configured so that fewer than all the cutting edges **112** are exposed at the same time. For example, the alignment may be such that when one cutting edge **112** is exposed by an opening **106,** the remaining cutting edges **112** are shielded within the housing **104.** Variations of such a configuration allow for any number of cutting edges to be exposed at any given time.

However, to even out the torque profile of the device when cutting, the cutter **108** is configured such that the number edges/cutting surfaces **112** of the flutes **110** that are aligned with the housing openings **106** does not vary throughout the rotational cycle. This prevents the catheter from being overloaded with torque spikes and cyclic torque variations due to multiple cutting edges/flutes engaging with tissue in synchrony. In other words, the length of the cutting surface **112** exposed through the openings **106** of the housing **104** remains the same or constant.

In the variation shown in Fig. 2B, the cutting surface **112** is configured to capture debris as it cuts. Typically, the device **100** may be designed with a secant effect. This effect allows for a positive tissue engagement by the cutter. As the cutter rotates through the opening, the cutting edge moves through an arc, where at the peak of the arc the cutting edge slightly protrudes above a plane of the opening. The amount of positive tissue engagement can be controlled through selection of the protrusion distance through appropriate design of the housing geometry (for example, by a combination of location and size of the window and radius of curvature of the housing). As shown, the cutting surface **112** extends out of the housing **104** through the window **106** as it rotates. This structure can also be designed to drive or impel the debris to the conveying member **118.** In this case, the flutes **110** within the cutter **108** are helically slotted to remain in fluid communication with the conveying member **118.** Variations of the device **100** can also include a vacuum source **152** fluidly coupled to the conveying member **118.** In order to improve the impelling force generated by the cutters, variations of the cutter have helical flutes **110** and sharp cutting edges **112** that are parallel to each other and are wound from proximal to distal in the same sense as the rotation of the cutter. When the cutter rotates, it becomes an impeller causing tissue debris to move proximally for evacuation.

As shown in Fig. 2C, variations of the device may have cutting surfaces **112** with positive rake angles **α-** that is the cutting edge is pointed in the same direction as that of the cutter rotation. This configuration maximizes the effectiveness of the impelling and cutting action (by biting into tissue and avoiding tissue deflection). The cutter is preferably made of hard, wear-resistant material such as hardened tool or stainless steels, Tungsten carbide, cobalt chromium, or titanium alloys with or without wear resistant coatings as described above. However, any material commonly used for similar surgical applications may be employed for the cutter. The outer surfaces of the proximal end of the cutter **108** are typically blunt and are designed to bear against the housing **104.** Typically, these surfaces should be parallel to the inner surface of the housing.

Figs. 2A-2B also show a surface of the cutter **108** having a curved-in profile distally and is close to the housing **104** surface. Note that housing slots **106** with this curved profile allows the cutting edge **112** to protrude beyond the housing's outer surface. In other words, the openings **106** form a secant on the curved surface of the housing **104.** Such a feature allows improved cutting of harder/stiffer material like calcified or stiff fibrous tissue where such tissue does not protrude into the housing **104.**

By controlling the number of cutting edges **112** that are exposed through openings **106** in the housing **104,** it is possible to control the relative amount of cutting engagement (both length of cutting and depth of cut, together which control the volume of tissue removed per unit rotation of the cutter). These features allow independent control of the maximum torque load imposed on the device **100.** By carefully selecting the geometry of the flutes and or cutting edges **112** relative to the openings **106** in the housing, it is possible to further control the balance of torque. For example, the torque load imposed on the device is caused by the shearing of tissue when the cutter edge is exposed by passing through the housing window. If all cutter edges simultaneously shear, as for example when the number of housing windows is an even multiple of cutter edges, the torque varies cyclically with rotation of the cutter. By adjusting the number of cutters and windows so one is not an even multiple of the other (for example, by using 5 windows on the housing and 4 cutting edges on the cutter), it is possible to have a more uniform torque (tissue removal from shearing action) during each cycle of the cutter.

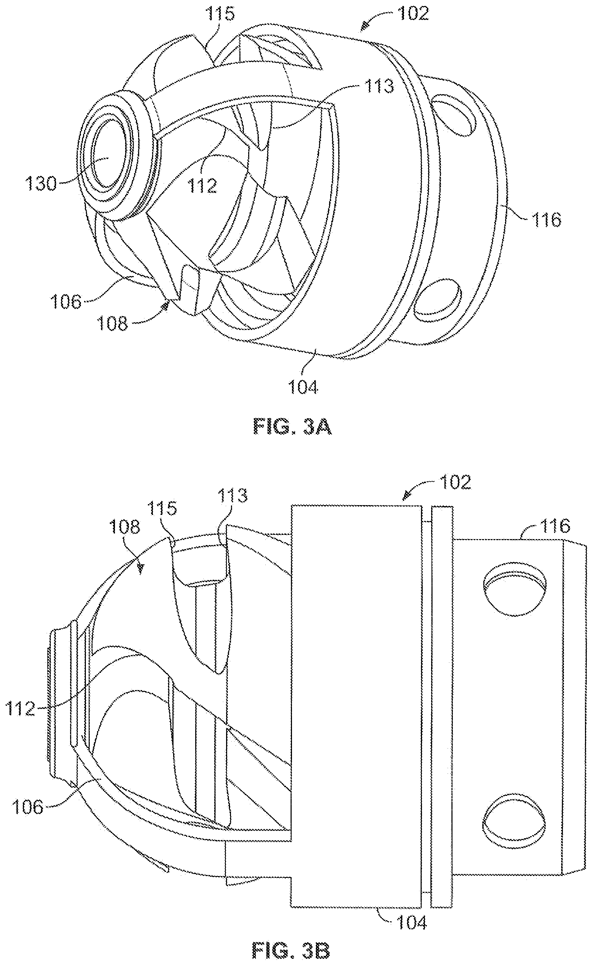

Fig. 3A illustrates another variation of a cutter assembly **102.** Fig. 3B shows a side view of the cutter assembly **102** of Fig. 3A. In this example, the cutting assembly **102** includes larger windows **106** to accommodate a cutter **108** that includes a plurality of directional cutting surfaces **112, 113, 115.** As the cutter **108** rotates within the housing **104,** the fluted cutting edge **112** cuts in a direction that is tangential to a rotational direction of the cutter **108.** In other words, the fluted cutting edges **112** cut material that is about the perimeter of the cutter **108** as it spins. The cutter **108** also includes on or more forward and rearward cutting surfaces **113, 115.** These cutting surfaces **113, 115** engage tissue when the catheter is run in a forward direction or rearward direction. The ability to engage and remove engagements in the multiple directions have been shown to be important for effective debulking. However, a variation of a cutter **108** in the present invention can include a cutter **108** with one or two directional cutting surfaces. For example, the fluted cutting edges **112** can be combined with either the forward **113** or rearward **115** cutting surfaces. The ability to debulk in a forward, rearward and rotational directions also reduces the chance that the cutter assembly deflects from stubborn or hard tissue.

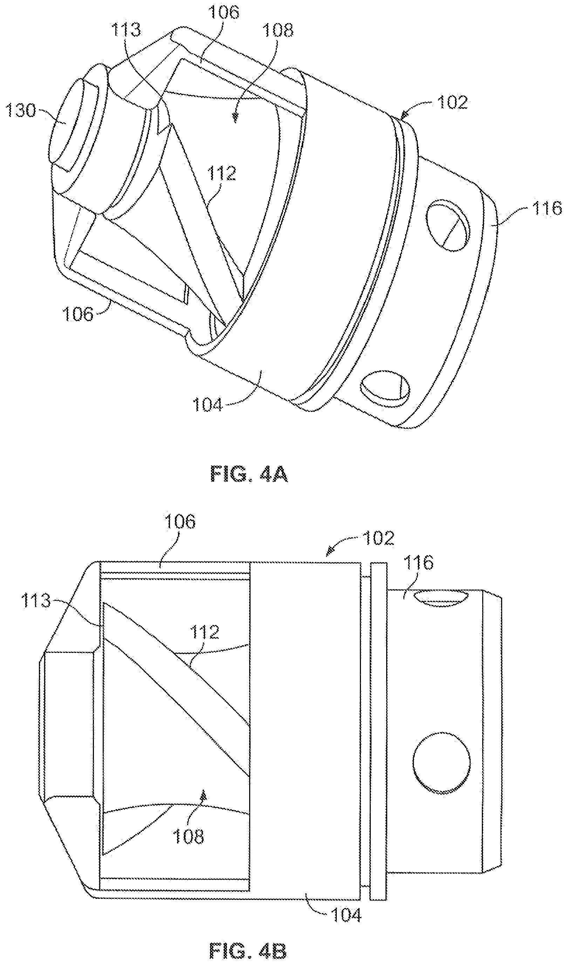

Figs. 4A and 4B show another variation of a cutter assembly **102** having a forward cutting surface **113** on a front of the cutter **108.** In this variation, the cutter housing **104** includes two large openings **106** that allow the forward cutting surface **113** to engage tissue when moved in a distal direction. The cutter **108** also includes a plurality of fluted cutting edges **112.**

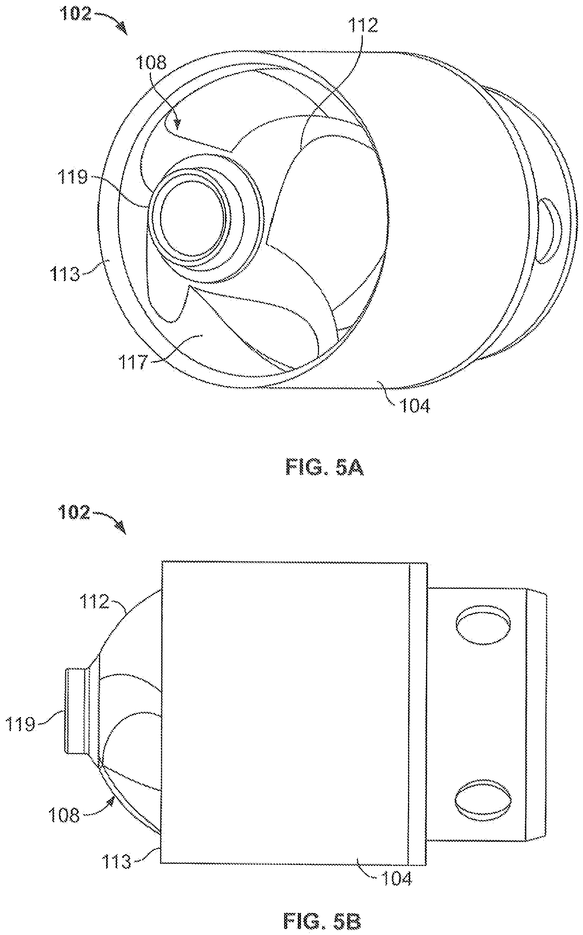

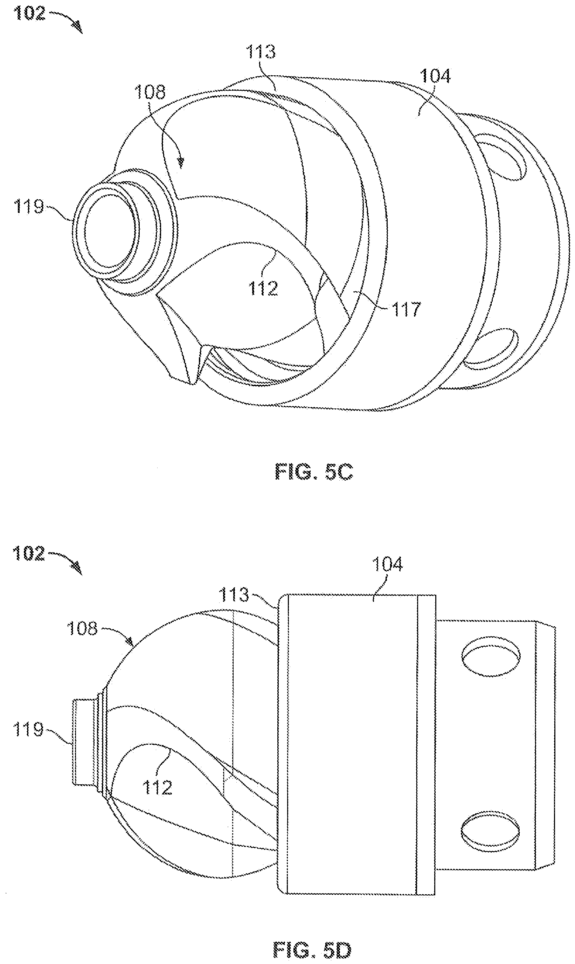

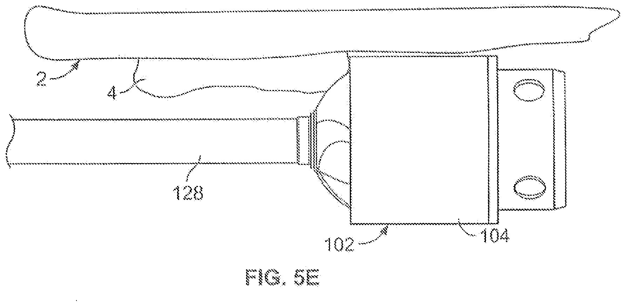

Figs. 5A and 5C show another variation of cutter assemblies **102.** In these variations, the cutter assemblies **102** include a cylindrical housing **104** containing a cutter **108** therein. The cutter **108** is exposed at a distal opening **117.** The edge 113 of the distal opening **117** forms a forward cutting surface. The housing **104** rotates along with the cutter **108** to assist in removal of tissue. As noted above, the forward cutting surface **113** engages and removes tissue or plaque **4** when the device is advanced in a distal direction within a body lumen **2** as shown in Fig. 5E. As discussed below, features of the device, including a guidewire **128** assist in preventing the device from excessively cutting the lumen wall **2.**

The housing **104** can be linked to the cutter **108** in a variety of ways as is well understood by those skilled in the art. For example the housing **104** can be directly linked or affixed to the cutter **108** so that both rotate together. Alternatively, the housing **104** can be geared to rotate faster or slower than the cutter **108.** In yet another variation, the gearing can be chosen to permit the housing **104** to rotate in an opposite direction than the cutter **108.**

Figs. 5B and 5D show respective side views of Figs. 5A and 5B. As shown, in Fig. 5B, the cutter **108** can protrude partially from the forward cutting surface **113** of the housing **104.** Fig. 5C shows a variation where the cutter **108** extends further from the housing **104** than the variation shown in Fig. 5B. Variations of the disclosure include cutters **108** that are totally recessed within the housing **108** or those having their entire fluted cutting edge **112** exposed distal to the cutting surface **113.** In any case, the fluted cutting edge **112** impels tissue debris back into the catheter. The outer diameter of the housing, proximal to the forward cutting surface **113** can be smooth to protect the lumen wall from the cutting action of the cutting edges. When the cutting assembly **102** is deflected, the outer diameter of the housing **102** becomes flush against the lumen wall and prevents the cutting edges from engaging the vessel wall (as shown in Fig. 5E). As the cutter assembly is advanced forward, it removes plaque **4** protruding from the lumen **2** wall and tissue debris is impelled backwards by the fluted edge **112** of the cutter **108.**

Figs. 5A to 5D also show a cutter assembly **102** having a blunt bumper **119** at the very tip of the cutter **108** that acts as a buffer to prevent accidental cutting into the guidewire or the vessel wall given the cutter assemblies' open distal design. In additional variations, the housing **104** could be expandable (such as a basket or mesh). As the cutter **108** gyrates inside the housing, the housing expands to cut a larger diameter.

The shielded atherectomy catheters described herein can perform biopsies, tumor removal, fibroid treatment, debulking of unwanted hyperplastic tissues such as enlarged prostate tissue, or other unwanted tissue such as herniated spinal disc material. The flexible, low profile catheter allows for ease of access to the treatment site and minimizes trauma or collateral damage to surrounding healthy tissue. With the continuous aspiration capability, contamination of the surrounding tissue during device introduction, treatment and removal is reduced or even eliminated. In addition, aspiration can be used to transfer biopsy tissue samples to outside the body for testing with the catheter remains in situ. This helps the physician make real time decision in advancing treatment of malignant tissue. The shield on the cutter assembly maintains controlled excision of tissue by limiting the depth of cutter engagement and thereby prevents the physician from inadvertently cutting into healthy surrounding tissue. The tip steering capability of the cutter allows the physician to direct the cutter towards desired site of tissue removal and minimizing collateral tissue damage. Finally, by deflecting the cutter and rotating the deflection to sweep in an arc, the catheter can excise large tumors or tissue lumps larger than the diameter of the catheter. Thus, excision of large tumors can be achieved through a small access channel and thereby minimizing trauma to the patient.

The construction of the cutting assembly can provide for additional modes of energy delivery. For example, the catheter excises tissue in vascularized regions excessive bleeding can occur (e.g., lung biopsy and excision). Accordingly, energy can be delivered to the target site via a conductive cutter assembly (i.e. shield or even cutter). Sound energy (ultrasound), electrical energy (radio frequency current), or even microwaves can be used for this purpose. These energy sources delivered through the cutter can also be used to denature tissue (collagen), shrink tissue, or ablate tissue.

The cutter assembly can be made from a variety of materials. For example, the housing is preferably made of a strong, wear resistant material such as hardened steels, cobalt chromium, carbides or titanium alloys with or without wear resistant coatings like TiNi. In particular the use of coatings will allow the use of tool steels which, unless coated, do not have acceptable corrosion resistance and biocompatibility. The cutter or cutter can be fabricated from steel and can be coated with a titanium nitride. Alternatively, the cutter can be fabricated from a tungsten carbide material.

Coatings can be applied to the moving components in the catheter to reduce friction. In one embodiment, the sheaths and the torque shaft are coating with a hydrophilic coating (polyvinyl alchohol) to reduce friction between the moving components in the catheter. The coatings can also be hydrophobic (e.g. parylene, PTFE). The coatings can be impregnated with heparin to reduce blood clotting on surface during use.

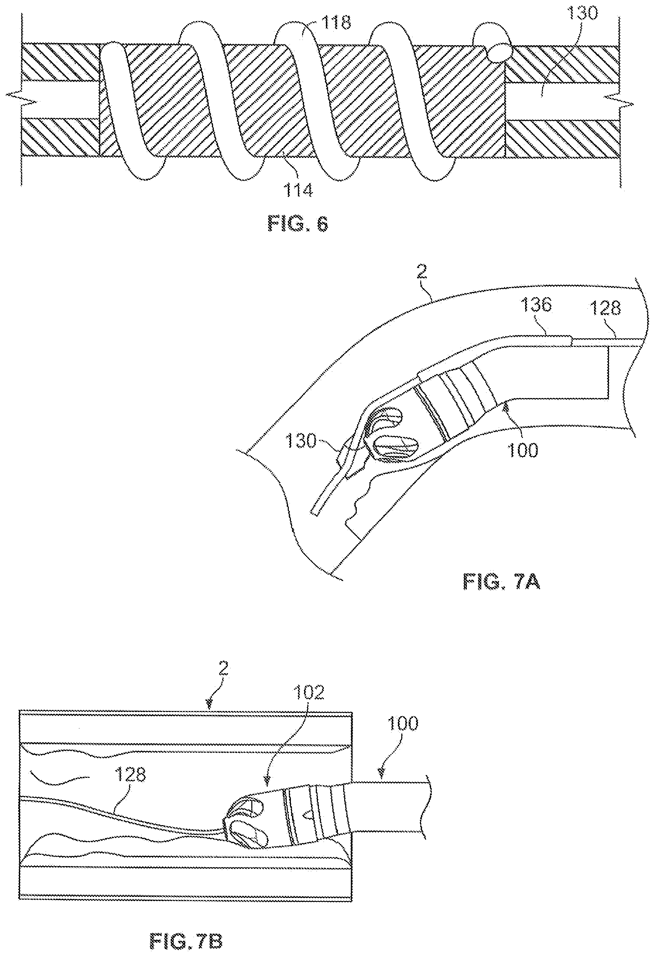

Fig. 6 shows a partial sectional view of an example of a torque shaft **114** that is coupled to a cutter assembly. To aid in removal of materials, the torque shaft can be a set of counter-wound coils, with the outer coil wound at the proper (greater) pitch to form the conveying member **118.** Winding the coils counter to each other automatically reinforces the torque shaft **114** during rotation. Alternatively, the torque shaft **114** may be made out of a rigid plastic, rendered flexible by incorporation of a conveying member **118.** Although the shaft may be fabricated from any standard material, variations of the shaft include a metal braid embedded in polymer (PEBAX, polyurethane, polyethylene, fluoropolymers, parylene) or one or more metal coils embedded in a polymer such as PEBAX, polyurethane, polyethylene, fluoropolymers or parylene. These constructions maximize torsional strength and stiffness, as well as column strength for "pushability", and minimize bending stiffness for flexibility. Such features are important for navigation of the catheter through tortuous vessels but allow for smooth transmission of torque over the long length of the catheter. In the multi-coil construction, the inner coil should be wound in the same sense as that of the rotation so that it would tend to open up under torque resistance. This ensures that the guidwire lumen remain patent during rotation. The next coil should be wound opposite the inner to counter the expansion to keep the inner coil from binding up against the outer catheter tube.

Fig. 6 also shows a torque shaft **114** having a central lumen **130.** Typically the lumen will be used to deliver a guidewire. In such cases, the central lumen may be coated with a lubricious material (such as a hydrophilic coating or Parylene) or made of a lubricious material such as PTFE to avoid binding with the guidewire. However, in some variations a guidewire section is affixed to a distal end of the housing. Moreover, the central lumen of the torque shaft **114** may also be used to deliver fluids to the operative site simultaneously with the guidewire or in place of the guidewire.

Fig. 7A illustrates a variation of a device **100** configured for rapid exchange. As shown, the device **100** includes a short passage, lumen, or other track **136** for the purpose of advancing the device **100** over a guidewire **128.** However, the track **136** does not extend along the entire length of the device **100.** Moreover, an additional portion of the track **136** may be located at a distal end of the catheter to center a guidewire **128.**

This feature permits rapid decoupling of the device **100** and guidewire **128** by merely holding the guidewire still and pulling or pushing the catheter **100** over the guidewire. One benefit of such a feature is that the guidewire **128** may remain close to the site while being decoupled from the device **100.** Accordingly, the surgeon can advance additional devices over the guidewire and to the site in a rapid fashion. This configuration allows for quick separation of the catheter from the wire and introduction of another catheter over the wire since most of the wire is outside of the catheter.

As shown in Fig. 7B, centering the tip of the cutting assembly **102** over a guide wire **128** improves the control, access and positioning of the cutting assembly **102** relative to a body lumen or vessel **2.** To accomplish this, the cutting assembly **102** can have a central lumen to accommodate a guide wire **128.** Variations of the device **100** includes a central guide wire lumen runs the length of the catheter through all central components including the torque shaft and the cutter. As noted above, a guidewire **128** can be affixed to the housing **104** or other non-rotational component of the cutting assembly **102.** In such a case, the guidewire **128** may preferably be a short segment that assists with navigation of the device through an occluded portion of a body lumen. However, the devices **100** can also operate without a guidewire since the head is steerable like a guidewire.

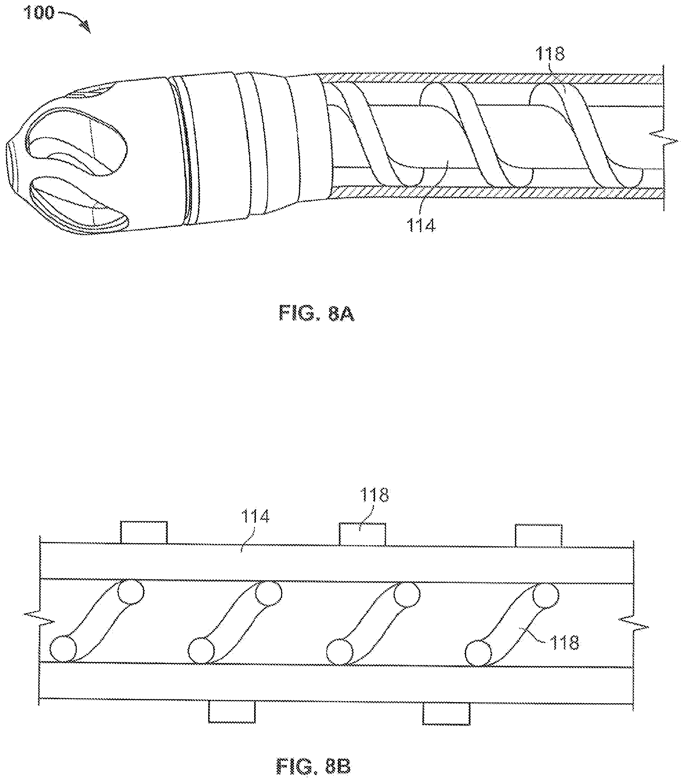

Fig. 8A illustrates a partial cross-sectional view of another variation of a device **100.** As shown, this variation of the device **100** includes a conveyor member **118** located within the device **100** and located on an exterior surface of a torque shaft **114.** The conveyor member **118** may be an auger type system or an Archimedes-type screw that conveys the debris and material generated during the procedure away from the operative site. In any case, the conveying member **118** will have a raised surface or blade that drives materials in a proximal direction away from the operative site. Such materials may be conveyed to a receptacle outside of the body or such materials may be stored within the device **100.** In one variation, the torque shaft **114** and conveying member **118** extend along the length of the catheter.

In some variations, the conveying member **118** may be integral to the shaft **114** (such as by cutting the conveying member **118** into the torque shaft **114** or by extruding the torque shaft **114** directly with a helical groove or protrusion. In an additional variation as shown in Fig. 8B, an additional conveying member **118** may be incorporated on an inside of the torque shaft, where the internal conveying member is wound opposite to that of the external conveying member **118.** Such a configuration allows for aspiration and debris (via the external conveying member **118)** and infusion (via the internal conveying member **118).** Such a dual action can enhance the ability to excise and aspirate plaque by: (1) thinning the blood, whether by viscosity alone or with the addition of anti-coagulants such as heparin or warfarin (cumadin), and/or anti-platetlet drugs such as Clopidegrel, (2) improving the pumpability (aspirability) of the excised plaque by converting it into a solid-liquid slurry that exhibits greater pumping efficiency, and (3) establishing a flow-controlled secondary method of trapping emboli that are not sheared directly into the housing, by establishing a local recirculation zone.

As noted above, the conveying member **118** can be wound in the same directional sense as the cutter **108** and in the same direction of rotation to effect aspiration of tissue debris. The impeller action of the cutter **108** moves the tissue debris from inside the housing **104** openings **106** into the torque shaft. The pitch of the cutting edges **112** may be matched in to that of the conveying member **118** to further optimize aspiration. Alternatively, the pitch of the conveying member **118** may be changed to increase the speed at which material moves once it enters the conveying member **118.** As discussed herein, debris can be evacuated outside the body by the conveying member **118** action along the length of the catheter and with or without supplement of the vacuum **152** pump connected to the catheter handle. Alternatively, the debris may be accumulated in a reservoir within the device.

The device may also include a ferrule **116,** as shown in Fig. 1B, that permits coupling of the catheter body **120**to the cutter assembly **102.** The ferrule **116** may serve as a bearing surface for rotation of the cutter **108** within the cutter assembly **102.** In the illustrated variation, the torque shaft **114** rotates inside the outer catheter body **120** and ferrule **116** to rotate the cutter and pull or aspirate tissue debris in a proximal direction. The clearance between the catheter tube and conveying member **118,** as well as the pitch and thread depth of the conveying member **118,** are chosen to provide the desired pumping effectiveness.

In one variation of the device, the housing **104** is connected to the catheter body **120** via the ferrule **116** and thus is static. The cutter **108** rotates relative to the housing **104** such that the cutting surface **112** on the cutter **108** shears or cleaves tissue and trap the tissue inside the housing **104** so that it can be evacuated in a proximal direction using the impeller action of the helical flutes and vacuum from the torque shaft. In alternate variations, such as where the housing includes a forward cutting surface, the housing **104** rotates as well as the cutter. Accordingly, the ferrule can serve as a bearing surface for both the housing and cutter.

The ferrule **116** can have a distal bearing surface to bear against the proximal surface of the cutter **108** and keeps the cutter axially stable in the housing **104.** In cases where the housing is stationary, the ferrule **116** can be rigidly bonded/linked to the housing **104** using solder, brazing, welding, adhesives (epoxy), swaging, crimped, press-fit, screwed on, snap-locked or otherwise affixed. As shown, the ferrule **116** can have holes or other rough features that allow for joining with the catheter body. While adhesives and heat fusing may be employed in the construction, such features are not required. Often adhesives are unreliable for a small surface contact and heat fusing can cause the tube to degrade. The use of a mechanical locking ring **126** allows the cutting assembly **102** to be short. Such a feature is important for maximizing the flexibility of the distal section of the catheter as it is required to navigate tortuosity in blood vessels.

In another aspect of the disclosure, devices **100** can be adapted to steer to remove materials that are located towards a side of the body passage. Such devices may include a deflecting member that permits adjusting the orientation or offset of the cutter assembly **102** relative to a central axis of the device. In Fig. 1B, the deflecting member comprises a catheter **122** with a sweep sheath deflecting member **132** (however, the deflecting member can be a tendon, wire, tube, mandrel, or other such structure.) As described herein, other variations are within the scope of the device.

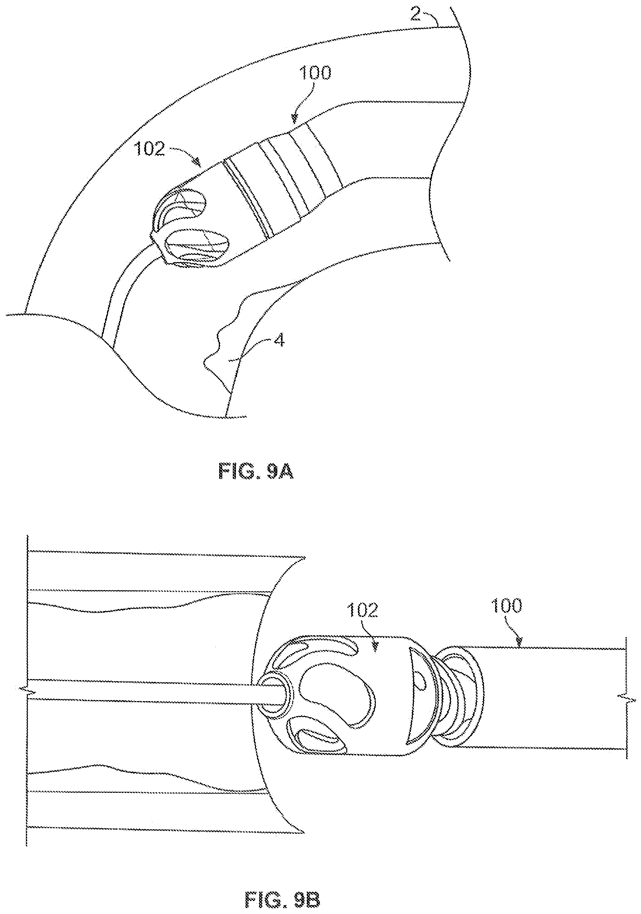

Fig. 9A illustrates an example of a variation of a device **100** equipped to have an articulating or steerable cutter assembly **102.** The ability to steer the tip of the device **100** is useful under a number of conditions. For example, when debulking an eccentric lesion as shown, the cutting assembly **102** should be pointed towards the side of the vessel **2** having the greater amount of stenotic material **4.** Naturally, this orientation helps prevent cutting into the bare wall/vessel **2** and focuses the cutting on stenotic tissue **4.** As shown in when in a curved section of the vessel **2,** without the ability to steer, the cutting assembly **102** would tend to bias towards the outside of the curve. Steering allows the cutting assembly **102** to point inward to avoid accidental cutting of vessel wall **2.**

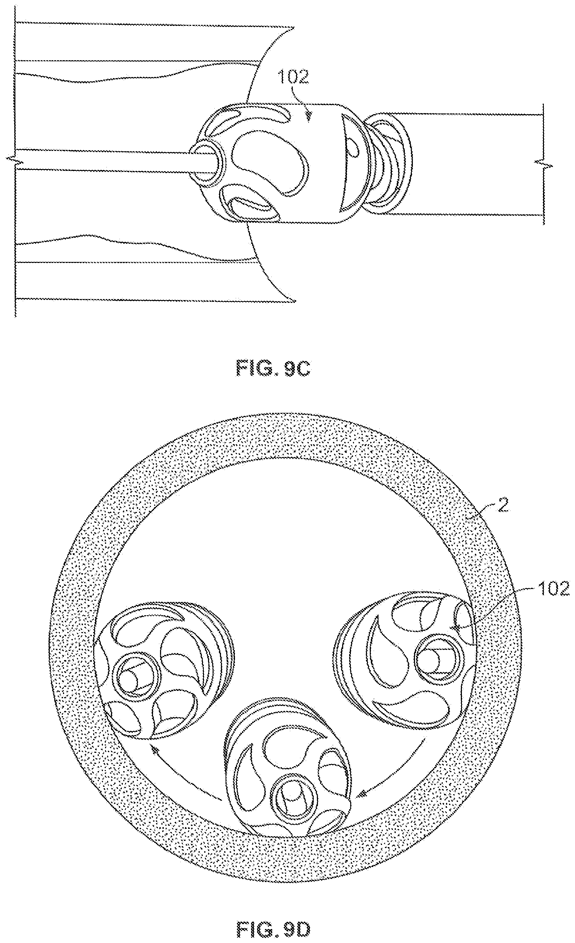

The ability to steer the device **100** also allows for a sweeping motion when cutting occlusive material. Fig. 9B shows the rotation of the cutting assembly **102.** As shown in Fig. 9C, when the cutting assembly **102** deflects relative to the axis of the catheter, rotation of the deflected portion **102** creates a sweeping motion. It is noted that rotation or articulation of the cutting assembly also includes rotation or articulation of the catheter to allow the cutting assembly to deflect relative to an axis of the catheter. Fig. 9D shows a front view taken along an axis of the vessel to illustrate the sweeping motion causing the cutting assembly **102** to "sweep" over a larger region than the diameter of the cutting assembly. In most cases, when articulated, the device will be rotated to sweep over an arc or even a full circle. The rotation of the cutter may or may not be independent of the rotation of the device. A user of the device may couple the sweeping motion of the cutting assembly with axial translation of the catheter for efficient creation of a larger diameter opening over a length of the occluded vessel. The combination of movement can be performed when the device is placed over a guidewire, for example by the use of a lead screw in the proximal handle assembly of the device. In another aspect of the devices described herein, the angle of articulation may be fixed so that the device sweeps in a uniform manner when rotated.

A number of variations to control the deflection of the device **100** are described herein. For example, as shown in Fig. 6 the sheath **122** itself may have a pre-set curve. In such a case, the area of the catheter body **120** adjacent to the cutting assembly **102** will be sufficiently flexible so as to assume the shape of the curved sheath **122.**

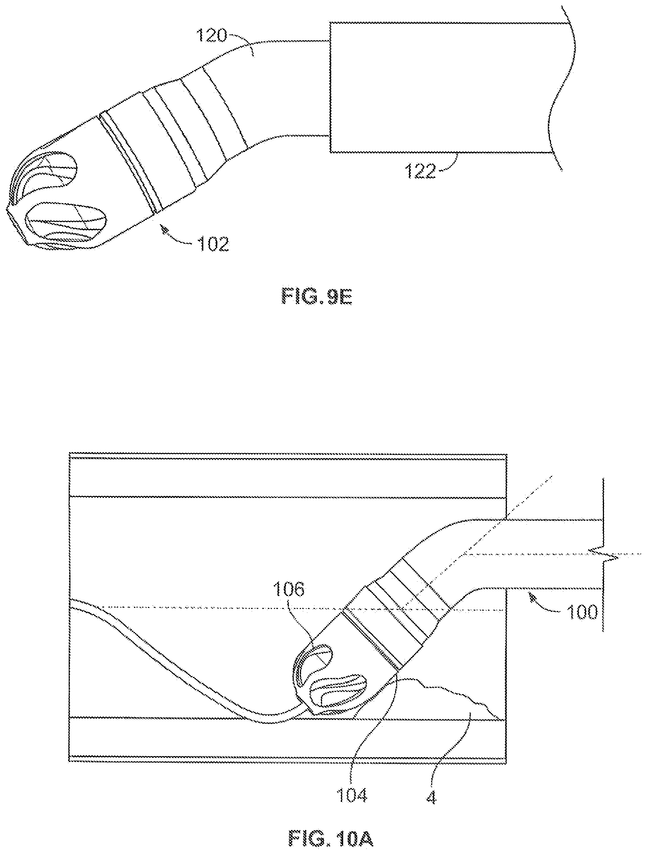

Fig. 9E illustrates another variation where the catheter body **120** includes a set curve in an area that is adjacent to the cutting assembly **102.** In this case, the outer sheath **122** can be made to be straight relative to the catheter body **120.** Accordingly, advancement of the curved portion of the catheter body **120** out of the sheath **122** causes the catheter body **120** to assume its curved shape. The degree of articulation in such a case may be related to the degree of which the catheter body **120** is advanced out of the sheath **122.**

In addition, the shape of the housing **104** as well as the location of the windows **106** can be chosen so that when the device **100** is substantially aligned with the lesion, or engages it at less than some critical attack angle, it will cut effectively. However, when pivoted at an angle greater than the critical angle, the cutting edges or grinding element will not engage the lesion as shown in Fig. 10A. This means that at large deflections, as the catheter tip approaches the vessel wall, it automatically reduces its depth of cut and ultimately will not cut when the critical angle is exceeded. For example, the cutter distal tip is blunt and does not cut. As the catheter tip is deflected outward, the blunt tip contacts the vessel and keeps the cutting edges proximal to the tip from contacting the vessel wall. Also the wire in combination with the device can also act as a buffer to prevent the cutting edges from reaching the vessel.

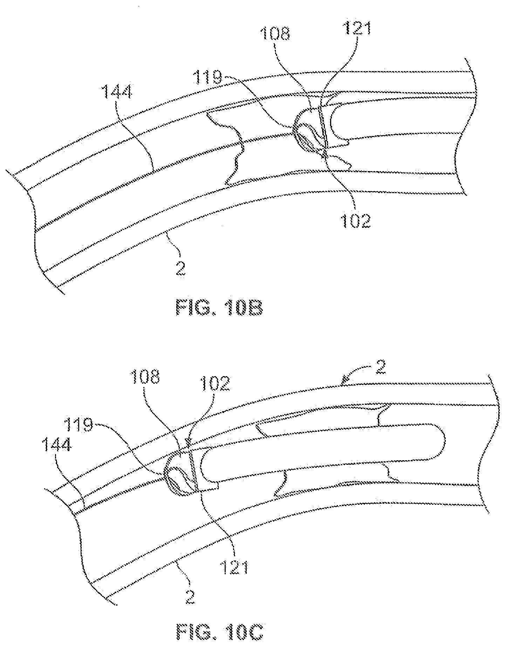

Figs. 10B and 10C show a cutter assembly design that is specialized for forward cutting. This particular variation includes an open ended housing where the cutter extends from the housing (as shown above). However, a blunt bumper **119** at the tip of the cutter **108** acts as a buffer to prevent accidental cutting into the guidewire **144** or excessively into the lumen wall **2.** In addition, this design can optionally incorporate a static housing portion **121** on a back end of the cutter assembly **102** that partially shields the cutter from deep side cuts into the lumen wall **2.**

As mentioned above, variations of the device **100** allow directional control of the cutting assembly **102.** In those variations where a slidable, torqueable sheath advances relative to the catheter body **122** (either external or internal to the catheter body) that can be flexed at the distal end. With the sheath flexed the catheter tip is pointed in the direction of the flex and the degree of bias is affected by the amount of flex on the sheath. The sheath can be rotated about the catheter or vessel long axis to change the direction of the cutting assembly. Also as noted above, this rotation can also effect a sweep of the cutting assembly **102** in an arc or a circle larger than a diameter of the cutter **102** (e.g. see Fig.9D). Such a feature eliminates the need to exchange the device for a separate cutting instrument having a larger cutting head. Not only does such a feature save procedure time, but the device is able to create variable sized openings in body lumens.

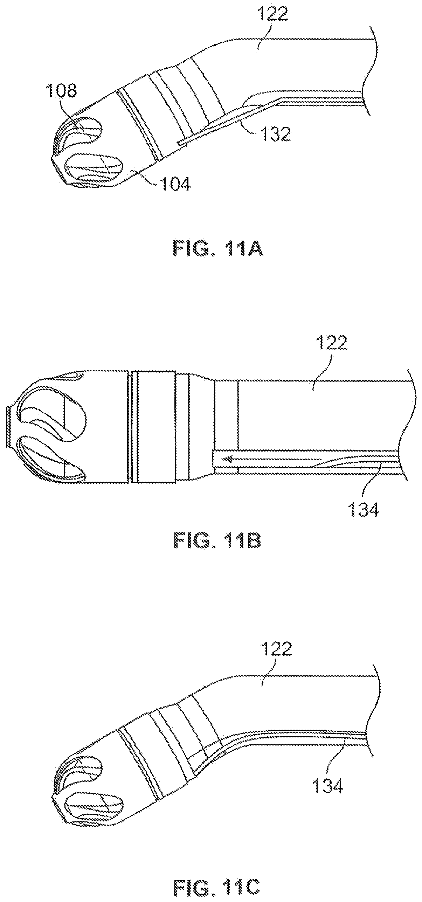

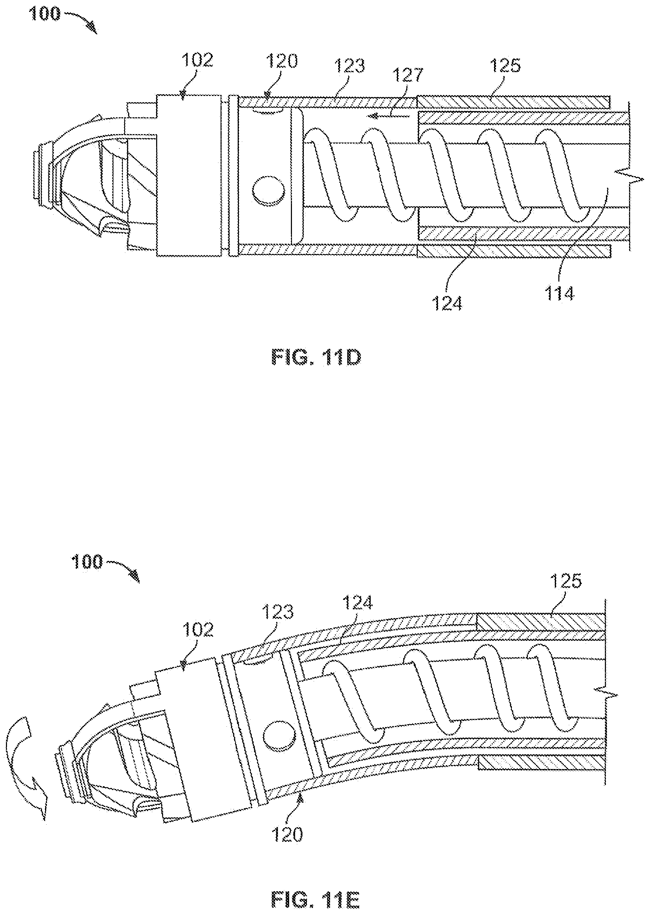

As shown in Fig. 11A, the tension on a slidable wire **132** in the wall of the sheath **122** can cause flexure of the sheath 122. Compression of the wire can also cause flexure of the sheath in the opposite direction. In one variation, the sheath **122** can be attached to the housing **104** of the cutting assembly **102.** Since the housing **104** is rotatable relative to the cutter **108** and the torque shaft **114,** the sheath **122** can rotate independently of the torque shaft **114** and cutter **108** to either sweep the cutting assembly **102** or to change direction of the articulated cutting assembly **102** at an independent rate.

In another variation of the device **100,** as shown in Fig. 11B, a preshaped curved wire or mandrel **134** can be advanced in a lumen in either the sheath **122** or catheter **120.** As the mandrel **134** advances, the device takes the shape as shown in Fig. 11C.

In yet another variation, the catheter tip and cutting assembly can be articulated in different directions and swept through an arc by having a series of sliding pull wires running through side lumens in the sheath. The pull wires attach to the cutter assembly. By cycling tension on the pull wires sequentially on the proximal control with such mechanism as a cam, the deflected tip can be swept in an arc.

Figs 11D to 11E illustrate a variation of a device **100** having a pre-shaped sweep sheath for a deflecting member **124** located in a space between the catheter **120** and the torque shaft **114.** In this variation, the catheter **120** includes a flexible distal portion **123** and a relatively stiffer proximal portion **125.** When the sweep sheath **124** is located within the stiffer proximal portion **125** of the catheter lumen the sweep sheath **124** straightens. To articulate the cutter assembly **102,** the operator advances the sweep sheath **124** as shown by arrow **127** such that the sweep sheath **124** locates within the flexible distal portion **123** of the catheter **120.** As a result, and as shown in Fig. 11B, the sweep sheath **124** causes articulation of the cutter assembly **102.** The sweep sheath **124** is rotatable as well as axially moveable within the catheter **124.** As a result, rotation of the sweep sheath **124** sweeps the cutter assembly **102** in an arc as discussed above.

As shown, the catheter body **120** remains stationary while the inner sweep sheath **124** rotates to move the cutting assembly **102** in an arc or orbit within the lumen. The outer catheter **120** body provides a static linkage between the cutter assembly and the deflection control assembly. The outer sheath is preferably composed of a metal braid sandwiched in a polymeric matrix of such materials as polyethylene (PE), fluoro-polymer (PTFE), nylon, polyether-block amide (PEBAX), polyurethane, and/or silicone. The sheath is stiffer proximally than distally. This can be achieved by using softer grades of polymers distally and/or having no metal braid distally.

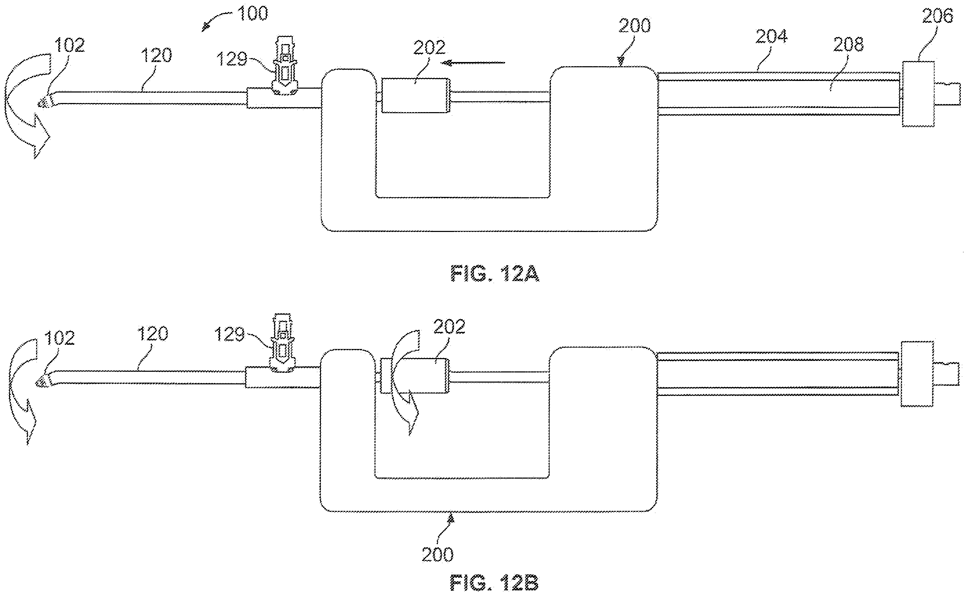

Fig. 12A and 12B illustrate one variation of a control system or fixture. As shown, the control system **200** includes a sweep control knob **202** coupled to a sweep sheath (not illustrated.) The sweep control knob **202** can slide axially and rotate independently relative to the outer catheter **120** and the torque shaft (not shown). Again, the sweep sheath can be composed of a metal braid sandwiched in a polymeric matrix of such materials as polyethylene (PE), fluoro-polymer (PTFE), nylon, and/or polyether-block amide (PEBAX), polyurethane, and/or silicone. The sweep sheath can also be made of counter wound metal coils. Its distal end is curved and is preferably made of a material that can withstand high degree of flex and retain its curved shape. Such material may include polymers such as PE, nylon, Polyetheretherketone (PEEK), Nickel Titanium (Nitinol), or spring steel.

To allow the cutter assembly to be straight and undeflected **102,** the sweep sheath is withdrawn proximally by the sweep control knob **202.** This causes the curved or shaped section of the sweep sheath to retract within the stiff portion of the outer catheter **120.** As shown in Fig. 12A, distal movement of the sweep control knob **202** advances the sweep sheath to deflect the catheter tip. The degree of the deflection is controlled by the amount the sweep sheath is advanced. The more the curve of the sweep sheath protrudes distal to the stiff section of the outer sheath, the more the catheter deflects.

As shown in Fig. 12B, the sweep control knob **202** can be rotated to sweep the cutting assembly **102** in an arc manner. Although sweeping of the cutting assembly **102** can occur via manual operation. Variations of the device include sweep sheaths that can be selectively coupled to a motor to activate an automated rotation. This allows the physician to have a smooth, continuous, automated means to sweep the cutter without any manual effort.

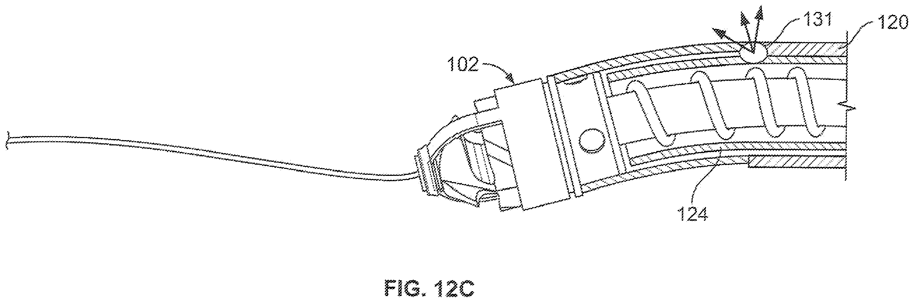

Figs. 12A and 12 B also show the catheter **120** as having a flush port **129.** The flush port **129** provides a means for injecting a fluid such as heparinized saline or any other medicine into the catheter body **120** to keep blood and tissue debris from clogging the space between components in the device. The flush port **129** can also help lubricate moving components within the device. One desirable fluid path is along the length of the catheter in the space between the catheter body **120** and sweep sheath **124.** Drugs or fluids can be introduced via the flush port **129** for flow out of one or more openings **131** near the catheter tip or cutting assembly **102.** In some variations, it may be desirable to place a flush opening **131** at an "elbow" of the catheter body **120** as shown in Fig. 12C. During use of the catheter the tip is deflected, the "elbow" always contacts the luminal surface. Drugs flushing out this elbow can then infuse into the vessel wall. Using a stenosis-inhibiting drug like paclitaxel or rapamycin could help prevent restenosis after the atherectomy procedure.

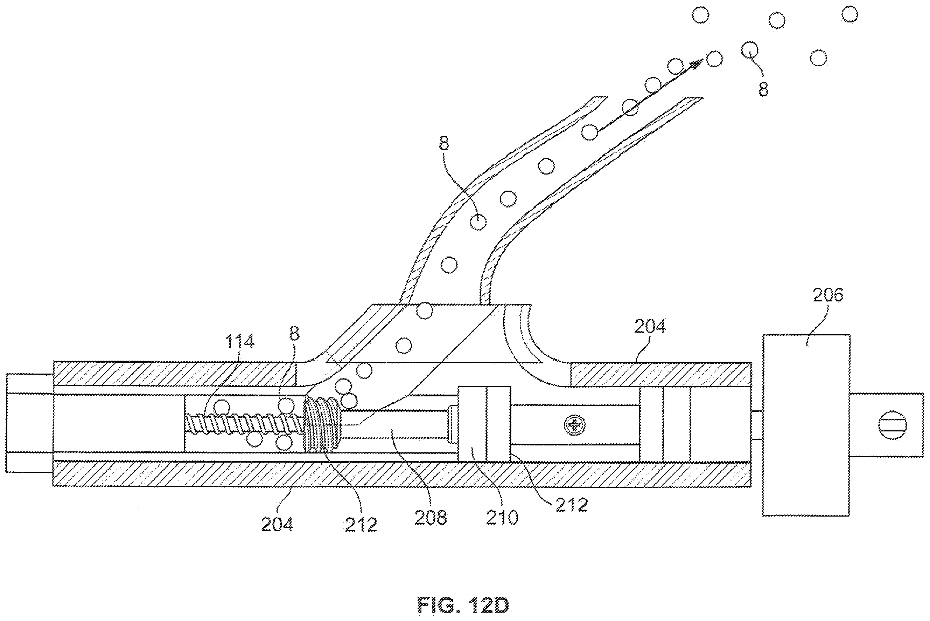

Turning now to a variation of the catheter **100** and control system **200,** the entire system is arranged from distal to proximal with a cutter assembly **102,** a catheter body **120** , a flush port **129,** a control system **200** for tip deflection and sweep control, a hub **204** or other connection for providing aspiration of the cut materials as well as a drive gear **206** to turn the torque shaft and cutter. The gear **206** is connected to a rigid drive shaft **208** encased within the hub **204** as shown in Fig. 12D. The drive shaft **208** can take a form of a hollow tube with a central lumen for passage of the guidewire and is centered within a lumen in the hub **204** and fixed axially by a pair of bearings **210.** A seal **212** adjacent to the bearing **210** prevents aspirated tissue debris from leaking proximally through the bearing **210.** A transfer propeller **212** is rigidly attached to the distal portion of the drive shaft **208** to pump aspirated tissue debris **8** from the catheter out into an attached aspiration reservoir. The drive shaft **208** is connected to flexible torque shaft **114** that extends the length of the catheter body for the purpose of transfer torque from the drive shaft to the cutter. As noted above, the torque shaft **114** has helical grooves on its outer diameter and central guidewire lumen. During a procedure run, a motor drives the gear **206** to rotate. This causes rotation of the drive shaft **208,** the transfer propeller **212,** the torque shaft **114,** and the cutter (not shown) all in the same rotational sense. Thus the cutter assembly effectively cuts plaque and drives the debris back into the helical groove on the torque shaft **114.** The rotating helical grooves winds the debris back into the hub **204,** which is then transferred to the aspiration reservoir by the transfer propeller **212.** The propeller **212** can take the form of a screw or a series of circumferentially arranged angled fan blades. The cutter is preferably rotated at a speed of 10,000-25,000 rpm. An alternative design would have the aspiration reservoir built into the hub of the catheter.

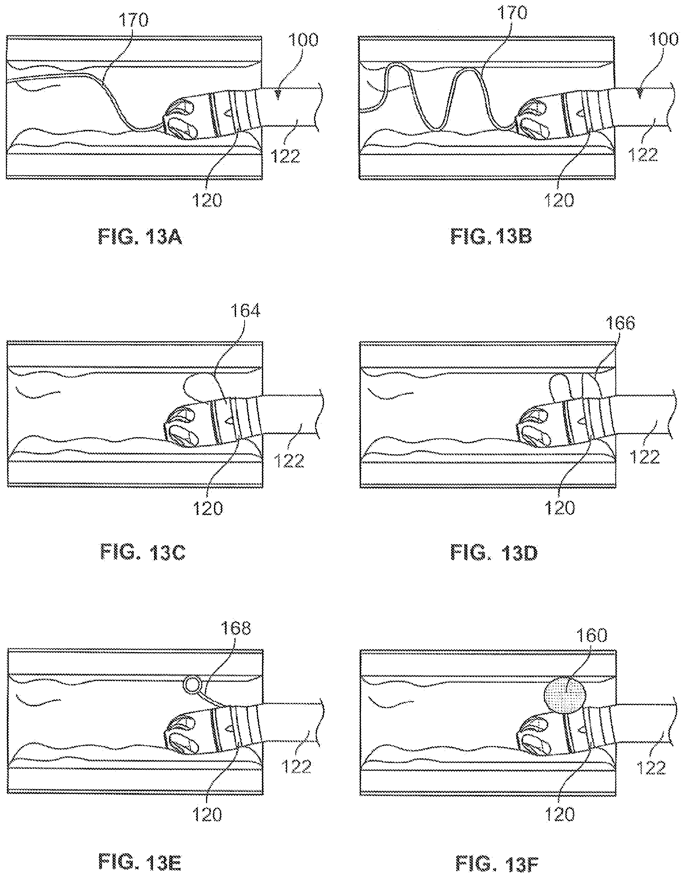

Figs. 13A to 13F illustrate additional mechanisms for flexing the device **100.** Such mechanisms can include side balloons **160,** meshes, wire loops **164,** coils **166,** and arms or mandrels **168** and other such structures. These features can be incorporated into catheter body **120** itself or into the sheath **122.** If located in the catheter body **122,** the entire catheter can be rotated to steer the tip in different directions. A curved or helical guidewire **170** can also be used to effect the flexion of the catheter tip as shown in Figs. 13A to 13F. The wire can also be actively flexed to control the degree of catheter flexion. All of these deflecting mechanisms can cause the catheter to be deflected in one plane or it can be deflected in three dimensions. The curve on the wire can be in one plane or in 3 dimensions. The sheath can be flexed in one plane or 3 dimensions. Another way to achieve flexion at the distal tip of the catheter is to only partially jacket the distal end with one or more polymers. A bevel at the distal end and/or varying combinations of jacketing and polymers can be used to change the position of the moment arm. This changes the flexibility of the distal end and allows proper deflection.

In addition to providing a means for deflecting the catheter, and allowing the user to sweep the distal tip to engage the lesion as desired, it is also possible to link a separate torque control device to manually or automatically control the sweep of the catheter, independent of the axial control of the catheter insertion and the rotation control of the cutter within the housing. Automatic control may be performed open-loop by user entered settings and activating a switch, or with feedback control designed to further optimize cutting effectiveness, procedural efficiency, and safety. Example structures of how to lock the articulation of the sheath/catheter into place include a lockable collar, a stopper, and friction lock detect mechanisms with one or more springs, coils, or hinges.

Additional components may be incorporated into the devices described herein. For example, it can be desirable to incorporate transducers into the distal region of the catheter to characterize the plaque or to assess plaque and wall thickness and vessel diameter for treatment planning; also transducers may be desired to indicate the progression of debulking or proximity of cutter to vessel wall. For example, pressure sensors mounted on the catheter housing can sense the increase in contact force encountered in the event that the housing is pressed against the vessel wall. Temperature sensors can be used to detect vulnerable plaque. Ultrasound transducers can be used to image luminal area, plaque thickness or volume, and wall thickness. Optical coherence tomography can be used to make plaque and wall thickness measurements. Electrodes can be used for sensing the impedance of contacted tissue, which allows discrimination between types of plaque and also vessel wall. Electrodes can also be used to deliver impulses of energy, for example to assess innervation, to either stimulate or inactivate smooth muscle, or to characterize the plaque (composition, thickness, etc.). For example, transient spasm may be introduced to bring the vessel to a smaller diameter easier to debulk, then reversed either electrically or pharmaceutically. Electrical energy may also be delivered to improve the delivery of drugs or biologic agents, by causing the cell membrane to open in response to the electric stimulation (electroporation). One method of characterization by electrical measurement is electrical impedance tomography.

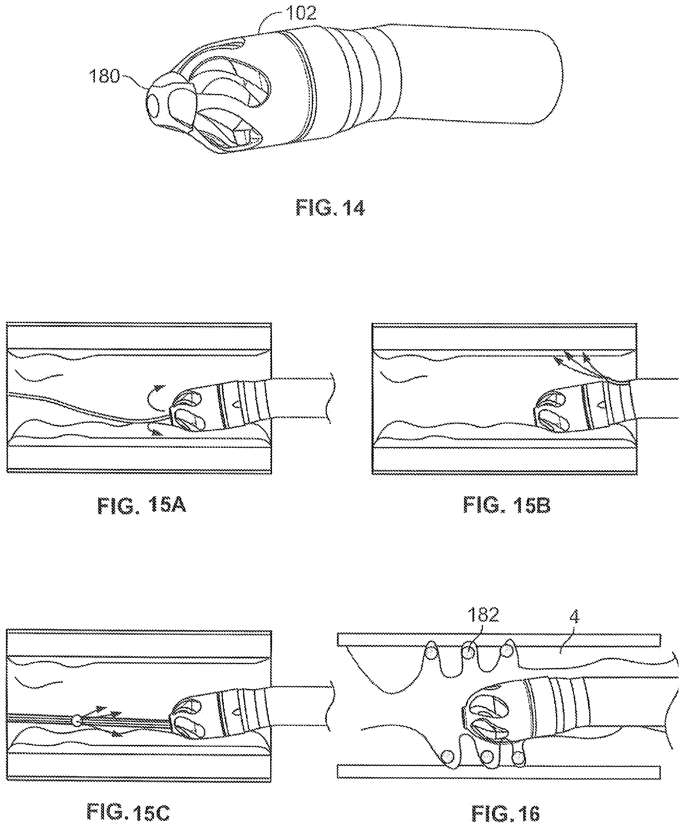

As shown in Fig. 14, a cutter assembly 102 can also have a burr protruding out its nose, in accordance with the present claimed invention. Although the burr **180** may have any type of abrasive surface, in one variation, this burr is blunt and has fine grit (such as diamond grit) to allow for grinding of heavily calcified tissue without injuring adjacent soft tissue. This combination of a burr and cutter allow the distal assembly to remove hard stenotic tissue (calcified plaque) using the burr while the sharp-edged shaving cutter removes softer tissue such as fibrous, fatty tissue, smooth muscle proliferation, or thrombus. In variations, the burr can also have helical flutes to help with aspiration, or the burr can be incorporated to a portion of the cutting edge (for example, the most distal aspect of the cutter).

Infusing solutions (flush) into the target treatment site may be desireable. Infused cool saline can prevent heating of blood and other tissue, which reduces the possibility of thrombus or othertissue damage. Heparinized saline can also prevent thrombus and thin out the blood to help maximize effectiveness of aspiration. The flush can also include drugs such as Clopidegrel, Rapamycin, Paclitaxel or other restenosis-inhibitors. This may help to prevent restenosis and may result in better long term patency. The flush may include paralytics or long-acting smooth muscle relaxants to prevent acute recoil of the vessel. Figs. 15A-15C illustrate variations of flushing out the device **100.** The flush can be infused through the guide wire lumen (Fig. 15A), a side lumen in the catheter shaft (Fig. 15B) or tube, the space between the flexing sheath and the catheter and/or the sideports in the guidwire (Fig.15C). Flush can come out of a port at the distal end of the cutter pointing the flush proximally to facility aspiration. Alternatively, by instilling the flush out the distal end of the cutter housing over the rounded surface, the flow may be directed rearward by the Coanda effect. The restenosis-inhibitors can be carried by microcapsules with tissue adhesives or vecro-like features on the surface to stick to inner vessel surface so that the drug adheres to the treatment site, and to provide a time-release controlled by the resorption or dissolving of the coating to further improve efficacy. Such velcro-like features may be constructed with nanoscale structures made of organic or inorganic materials. Reducing the volume of foreign matter and exposing remaining tissue and extracellular matrix to drugs, stimulation, or sensors can make any of these techniques more effective.

Another way to infuse fluid is to supply pressurized fluid at the proximal portion of the guidewire lumen (gravity or pressure feed) intravenous bag, for example. A hemostatic seal with a side branch is useful for this purpose; tuohy-borst adapters are one example of a means to implement this.

Balancing the relative amount of infusion versus fluid volume aspirated allows control over the vessel diameter; aspirating more fluid than is instilled will evacuate the vessel, shrinking its diameter, and allow cutting of lesion at a greater diameter than the atherectomy catheter. This has been a problem for certain open cutter designs that use aspiration, because the aggressive aspiration required to trap the embolic particles evacuates and collapses the artery around the cutter blades; this is both a performance issue because the cutter can bog down from too high torque load, and the cutter can easily perforate the vessel. The shielded design described here obviates both problems, and further requires less aggressive aspiration to be effective, giving a wider range of control to the user.

The devices of the present invention and disclosure may also be used in conjunction with other structures placed in the body lumens. For example, as shown in Fig. 16, one way to protect the vessel and also allow for maximum plaque volume reduction is to deploy a protective structure such as a stent, thin expandable coil or an expandable mesh **182** within a lesion. As this structure expands after deployment, the thin wire coil or the struts push radially outward through the plaque until it becomes substantially flush with the vessel wall. This expansion of thin members requires minimal displacement of plaque volume and minimizes barotrauma produced in balloon angioplasty or balloon expanded stent delivery. Once the protective structure has expanded fully, atherectomy can be performed to cut away the plaque inside to open up the lumen. The vessel wall is protected by the expanded structure because the structure members (coil or struts) resist cutting by the atherectomy cutter, and are disposed in a way that they cannot invaginate into the cutter housing (and thereby be grabbed by the cutter). It is also possible to adjust the angle of the windows on the atherectomy catheter cutter housing so that they do not align with the struts or coils; the adjustment to orientation may be accounted for in the coil or strut design, in the cutter housing design, or both. Furthermore, the protective member can be relatively flexible and have a low profile (thin elements), so that it may be left in place as a stent. Because the stent in this case relies mainly upon atherectomy to restore lumen patency, it may be designed to exert far less radial force as it is deployed. This allows usage of greater range of materials, some of which may not have as high of stiffness and strength such as bioresorbable polymers and metal alloys. Also, this allows a more resilient design, amenable to the mechanical forces in the peripheral arteries. It also minimizes flow disruption, to minimize hemodynamic complications such as thrombosis related to the relatively low flows found in the periphery. It is also possible to perform atherectomy prior to placing the protective structure, whether or not atherectomy is performed after placing the structure.

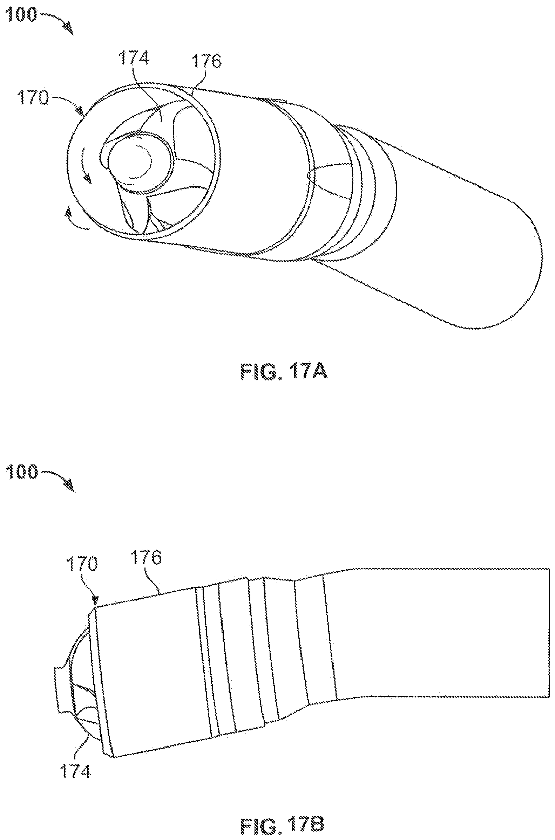

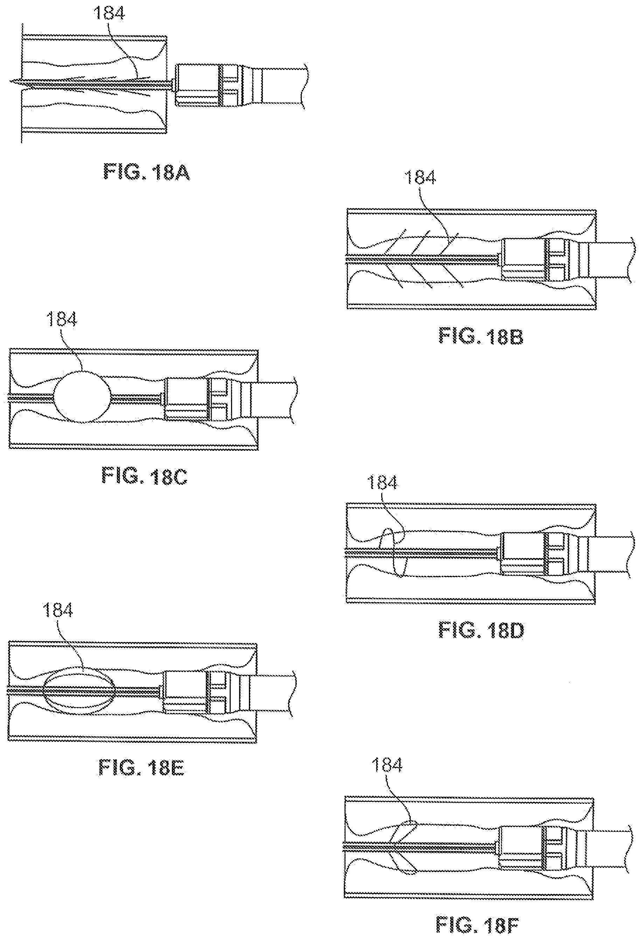

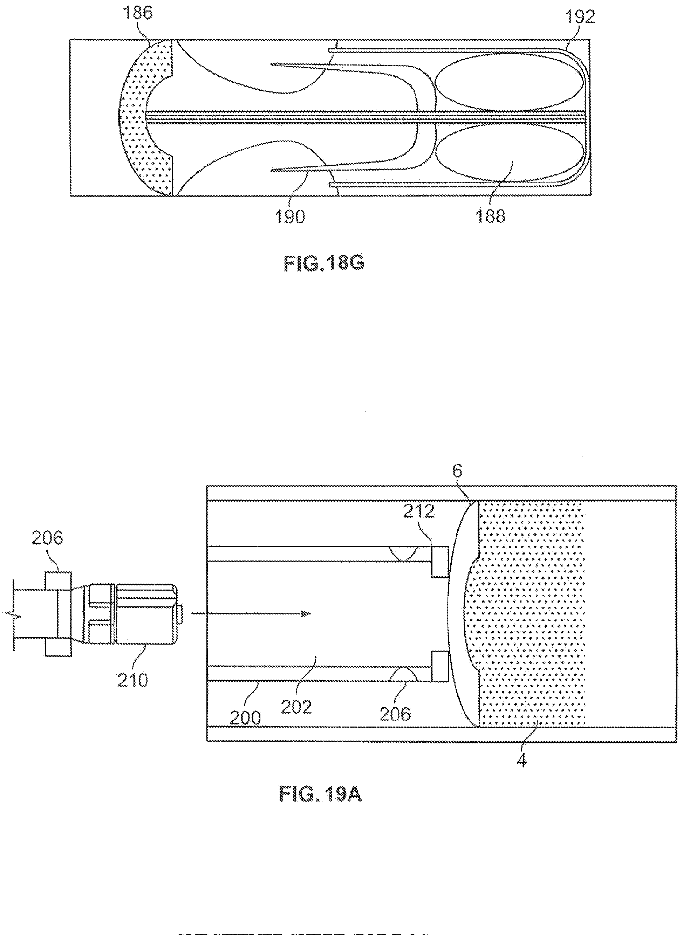

Additional variations of systems include devices **100** having a cutting assembly **170** comprising spinning turbine-like coring cutter **172** as shown above and as shown in Fig. 17A. Fig. 17B shows a side view of the coring cutter **170.** In use, the coring cutter can be hydraulically pushed to drive the sharp edge through tissue. The turbine like cutters has helical blades **174** on the inside of the sharp cylinder housing **176** (shell). The coring cutter **170** may also have spokes or centering devices **184** as shown to in Figs. 18A to 18F center the shell about the guidewire. This helps to keep the cut of the plaque centered about the vessel wall for safety. The spokes also act as an impeller to pull stenotic tissue back and this helps to drive the cutter forward as well as achieve aspiration to minimize embolization. In the hydraulically driven cutter design, an anchor **186** is deployed in tissue and is connected to a backstop **192.** A balloon or hydraulic chamber **188** is then pressurized to expand and pushes the cutting blade **190** forward through the lesion (See Fig. 18G ). One advantage of this approach may be that the technique is similar to angioplasty (which involves pumping up a balloon with an endoflator). One means of anchoring is to use an anchoring guidewire, for example, a guidewire with an inflatable balloon to be placed distal to the atherectomy catheter. Alternatively, the technique of anchoring distally can be used with the previously described torque shaft driven atherectomy catheter.

It is also possible to use the devices and exemplary methods described here to restore patency to arterial lesions in the coronary circulation and in the cerebrovalscular circulation, both by debulking de novo lesions and by debulking in stent restenosis.

The devices and exemplary methods described herein also work particularly well in lesions that are challenging to treat with other methods: at bifurcations, in tortuous arteries, and in arteries which are subject to biomechanical stresses (such as in the knee or other joints).

In a further variation of the devices described here, the motor drive unit may be powered by a controller that varies the speed and torque supplied to the catheter to optimize cutting efficiency or to automatically orbit the cutter using variable speed with a fixed flexible distal length of catheter (or providing further orbiting control by controlling the length of the distal flexible section of the catheter).

It is also possible to use feedback control to operate the catheter in a vessel safe mode, so that the rate of cutting is decreased as the vessel wall is approached. This may be accomplished through speed control, or by reducing the degree to which the cutting blades penetrate above the housing window by retracting the cutter axially within the housing. Feedback variables could be by optical (infrared) or ultrasound transducer, or by other transducers (pressure, electrical impedance, etc.), or by monitoring motor performance. Feedback variables may also be used in safety algorithms to stop the cutter, for example in a torque overload situation.

The atherectomy catheter may be further configured with a balloon proximal to the cutter, for adjunctive angioplasty or stent delivery. The catheter may optionally be configured to deliver self-expanding stents. This provides convenience to the user and greater assurance of adjunctive therapy at the intended location where atherectomy was performed.

Further exemplary methods include use of similar devices to debulk stenosis in AV hemodialysis access sites (fistulae and synthetic grafts), as well as to remove thrombus. By removing the cutter housing and recessing the fluted cutter within the catheter sheath, a suitable non-cutting thrombectomy catheter may be constructed.

Other exemplary methods of use include excising bone, cartilage, connective tissue, or muscle during minimally invasive surgical procedures. For example, a catheter that includes cutting and burr elements may be used to gain access to the spine for performing laminectomy or facetectomy procedures to alleviate spinal stenosis. For this application, the catheter may be further designed to deploy through a rigid cannula over part of its length, or have a rigid portion itself, to aid in surgical insertion and navigation.

For this reason, it is advantageous to couple atherectomy with stenting. By removing material, debulking the lesion, a lesser radial force is required to further open the artery and maintain lumen diameter. The amount of debulking can be tuned to perform well in concert with the mechanical characteristics of the selected stent. For stents that supply greater expansion and radial force, relatively less atherectomy is required for satisfactory result. An alternative treatment approach is to debulk the lesion substantially, which will allow placement of a stent optimized for the mechanical conditions inherent in the peripheral anatomy. In essence, the stent can support itself against the vessel wall and supply mild radial force to preserve luminal patency. The stent may be bioresorbable, and/or drug eluting, with the resorption or elution happening over a period for days to up to 12 weeks or more. A period of 4 to 12 weeks matches well with the time course of remodeling and return to stability as seen in the classic wound healing response, and in particular the known remodeling time course of arteries following stent procedures. In addition, the stent geometry can be optimized to minimize thrombosis by inducing swirl in the blood flow. This has the effect of minimizing or eliminating stagnant or recirculating flow that leads to thrombus formation. Spiral construction of at least the proximal (upstream) portion of the stent will achieve this. It is also beneficial to ensure that flow immediately distal to the stent does not create any stagnant or recirculation zones, and swirl is a way to prevent this also.

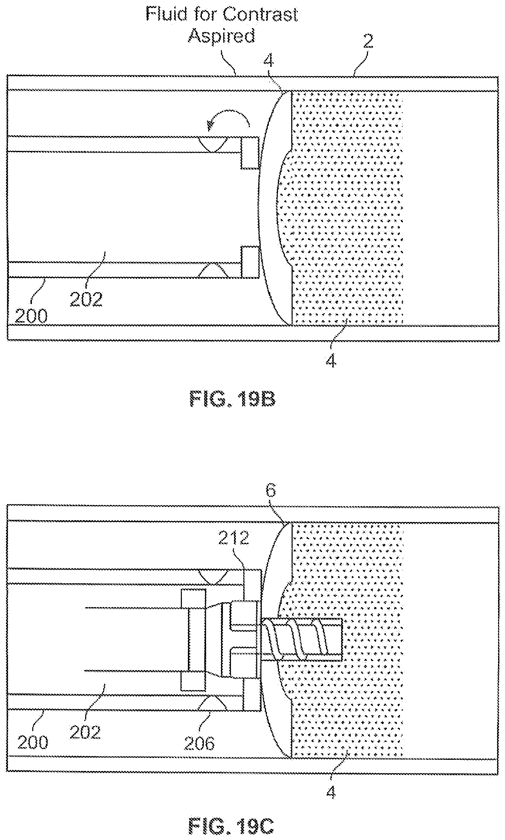

Fig. 19A illustrates another variation of a device for clearing obstructions within body lumens. In some cases where a vessel is totally occluded, a tough fibrous or calcific cap 6 completely or almost completely blocks the lumen. Because of this blockage, fluid cannot flow past the occlusion. This stagnation also makes it difficult or impossible to properly insert a wire across the lesion with an atherectomy device or stiff catheter.

In a typical case of a total occlusion, it is also difficult if not impossible to visualize the lumen near the occlusion because any injected contrast agents cannot flow through the occlusion site.

Fig. 19A shows a system for treating total occlusions. The system can include a support catheter comprising a support tube or catheter **200,** having a central lumen **202,** the catheter may include side lumens or ports **206,** for flush and aspiration. The catheter central lumen **202** can be used to deliver contrast agents **208.** In addition, tip centering mechanisms, and an atraumatic tip can be useful. The support catheter can be used with any lumen-creating device **210,** such as the devices **100** described above, a laser catheter, an RF probe, or an RF guidewire. When using a coring cutter as shown in Fig. 19A, the cutter can have a sharp edge at its tip, helical flutes, helical grooves, or any other mechanism that enables penetration of the fibrous or calcific cap. The cutter and the shaft can be advanced forward within the support catheter, and one or more balloons or baskets can also be deployed by the support catheter to help center it in the vessel.

The lumen-creating device **200** can optionally be made to have a shoulder **212** at its distal end, as shown in Figure 19A. The shoulder **212** acts as a stop to limit the depth at which the device **200** protrudes beyond the support catheter **200.** Such a safety measure may be desired to protect the vessel wall. Driving the device **200** through the tough fibrous cap creates a lumen in the cap. A guidewire may then be placed into the lumen created in the fibrous cap. The coring cutter may be removed with the core.

Next, a guidewire can be used with a cutter assembly to remove some or all of the remaining mass in the vessel. Alternatively, the initial lumen made may be adequately large without further atherectomy. Technical success is typically less than 30 percent or less than 20 percent residual stenosis. Also, balloon angioplasty with or without stenting may be performed following establishment of a guidewire lumen with a support catheter and a lumen-creating catheter.

Contrast injection and aspiration ports near the distal end of the support circulate contrast agents, enabling the use of fluoroscopy to visualize the lumen adjacent to the total occlusion during diagnosis or treatment. The central lumen **202** of the support catheter **200** can also be used to inject or aspire the contrast agents **208.** The contrast agents can circulate through the center lumen **202** in the support catheter **200** and at least one port **206** in various configurations. The fluid can circulate about the distal tip of the catheter, the motion of the fluid being circular as shown in Fig. 19B. For example, the fluid can be injected through the central lumen **202,** travel around the distal tip, and then is aspirated back into the support catheter through ports **206** on the side of the surface of the support catheter **200.** To illustrate another possible configuration, the fluid can be ejected through the side ports, and then aspired through the central lumen. This recirculation of the contrast agent permits imaging of the vessel at the site of the occlusion.

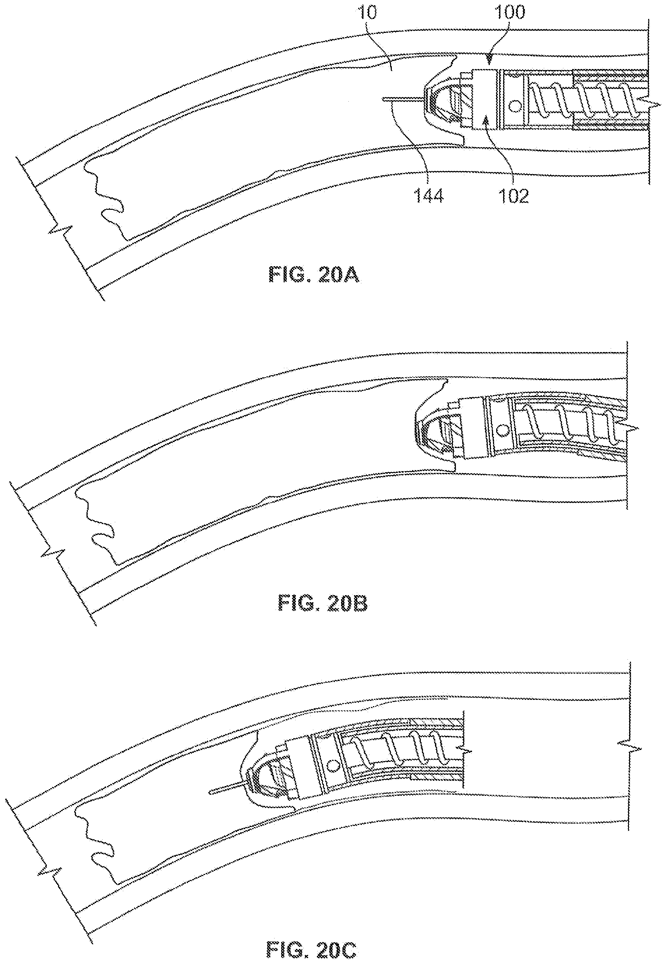

Any of the atherectomy devices **100** described herein can be used as a tool to treat chronic total occlusions (CTO) or a complete blockage of the artery. The frontward cutting and tip-steering capabilities allows the physician to controllably create a channel through the blockage. In one such method for creating this channel (recanalization) the physician places the device **100** proximal edge of a blockage **10** as shown in Fig. 20A.

The physican steer the cutting assembly **102** tip towards the center of the vessel as described above. Then, the physician advances a guidewire **144** forward to penetrate the blockage **10.** Now that the cutting assembly **102** is located in or adjacent to the blockage **10,** the motor is actuated to begin the cutting process allowing the cutting assembly **102** to follow the guidewire **114.** During this process, the physician steers the cutting assembly **102** or catheter tip as necessary to keep the cutter centered in the lumen. With the catheter support close to its tip, the wire can be controllably advanced further through the blockage and the catheter can follow by cutting its way forward as shown in Fig. 20C.

The process continues until the cutting assembly **102** passes through the blockage **10.** However, during the recanalization process, the guidewire **144** can be exchanged easily, as shown in Fig. 20B, so that the physician can selectively use the optimal guidewire. The catheter has a guidewire lumen that runs along its length and thus allows for this guidewire exchange.

Typically, the physician is not able to visualize the anatomy adjacent to the blockage using the fluoroscope because contrast dye injection cannot be performed due to the complete blockage. The catheter above overcomes this problem because of its ability to aspirate at the cutting assembly. Thus dye injection can be introduced into the target area and circulated back into the catheter via the catheter aspiration mechanism. The flow of dye near the catheter tip allows the physician to visualize the anatomy during the recanalization process.

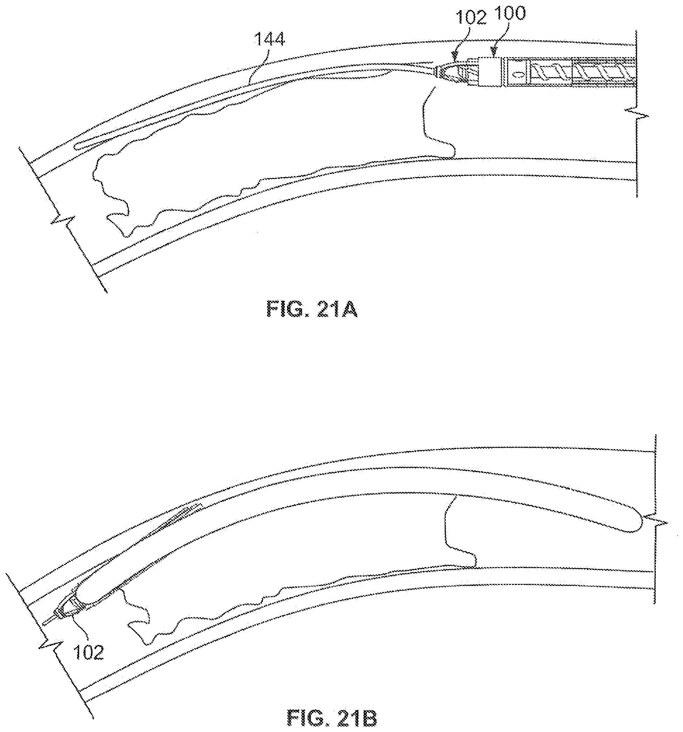

Figs. 21A and 21B show another exemplary method of recanalizing a blockage **10.** In this variation, the physician advances the guidewire **144** through the device **100** and between the tissue layers of the vessel (subintimal channel) such as the space between the tunica media (middle layer of vessel wall) and the tunica adventitia (outer elastic layer of the vessel wall). The atherectomy device **100** described herein can be used to provide support for the advancement of the guidewire **144** through the subintimal channel as well as a means for steering back into true lumen. Once the wire has crossed the blockage through the subintimal channel, the device advances forward to follow the guidewire **144** across the blockage **10** and is steered back torwards the true lumen as shown in Fig. 21B. The cutter assembly **102** can be activated to help pierce a channel into back into the true lumen. In clinical terminology, this catheter is used as a "re-entry device".

The deflected catheter tip is elastic or spring-like. The degree of the deflection is limited by the diameter of the lumen. As plaque is removed during the atherectomy process, the degree of deflection automatically increases. Since the deflected catheter tip is radiopaque (can be visualized with fluoroscope), its degree of deflection can be continuously visualized during the procedure and thus allows the physician to visualize the progress in the opening of the lumen without having to pause and perform a dye injection. Limiting dye injections helps to minimize health problems for the patient and saves procedure time.

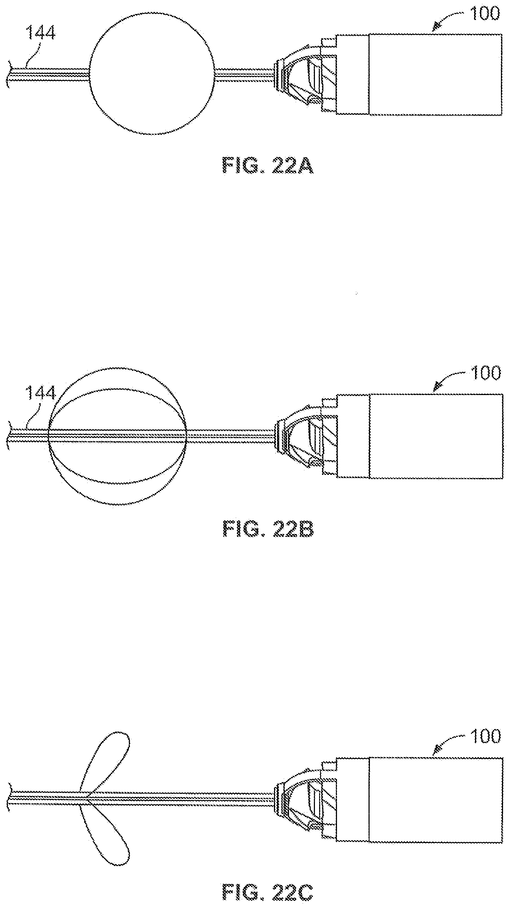

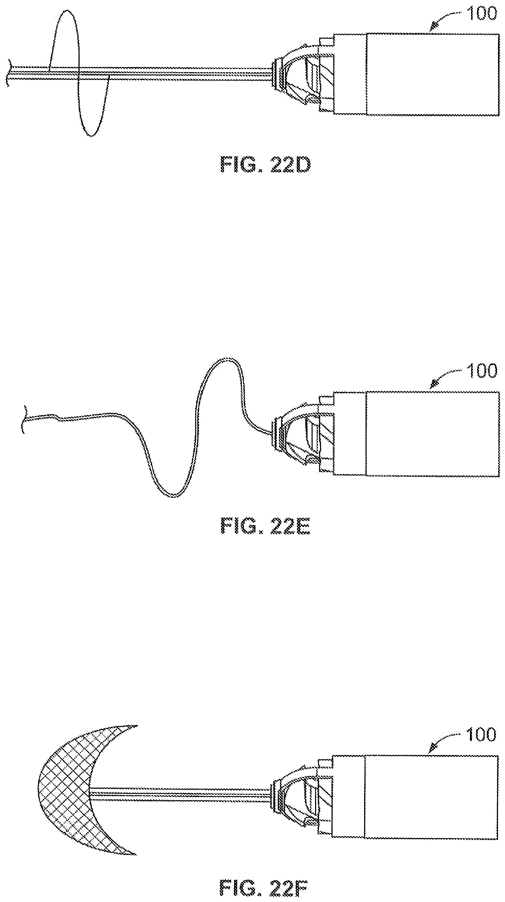

It is important to ensure that the guidewire is axially fixed relative to the target vessel during operation of the atherectomy device **100** to prevent the guidewire tip **144** from traumatizing the vessel. This can be accomplished by having an anchoring mechanism **154** to anchor the wire to the vessel. The mechanism **154** can be an inflatable balloon on the wire as shown in Fig. 22A, expandable basket as shown in Fig. 22B, deployable loops as shown in Fig. 22C, a deployable helical coil Fig. 22D, a wire taking on a shape of a helical coil or a wave on the guidewire Fig. 22E, a porous umbrella or a mesh as shown in Fig. 22F. These mechanisms can all collapse compactly onto profile as small as a typical guidewire to pass through the guidewire lumen on the catheter and expand as large as the distal vessel diameter to anchor. These mechanisms can also act as a way to prevent plaque debris from floating down stream to provide a type of embolic protection.

It is noted that the descriptions above are intended to provide exemplary embodiments of the devices and exemplary methods. It is understood that, the invention includes combinations of aspects of embodiments or combinations of the embodiments themselves. Such variations and combinations are within the scope of this disclosure. The invention is defined by the following claims.

## Claims

1. A device (100) for removing material from body lumens, the device comprising:
a catheter body (120) having a proximal end, a distal end and a catheter lumen extending therethrough;
a cutter assembly (102) located at the distal end of the catheter body, the cutter assembly comprising:
a housing (104) having at least one opening (106);
a cutter (108) configured to rotate within the housing, the cutter including at least one fluted cutting edge (112) that is configured to cut in a direction that is tangential to the rotational direction of the cutter;
a torque shaft (114) extending through the catheter lumen and having a first end coupled to the cutter assembly and a second end adapted to couple to a rotating mechanism (150), wherein an exterior surface of the torque shaft comprises a raised surface helically located thereon and adapted such that upon rotation, the raised surface conveys materials in a proximal direction, **characterised by** a burr (180) protruding distal of the cutter assembly.

2. The device (100) of claim 1, further comprising a tube member linking the housing (104) to a distal end of the catheter body (120).

3. The device (100) of claim 1, wherein the cutter (108) is cylindrically shaped, a proximal portion of the housing (104) is cylindrical and a distal portion of the housing is tapered.

4. The device (100) of claim 1, wherein the burr (180) has abrasive surface.

5. The device (100) of claim 1, wherein the burr (180) is blunt and has fine grit.

6. The device (100) of claim 5, wherein the fine grit is diamond grit.

7. The device (100) of claim 1, wherein the burr (180) has helical flutes for assisting aspiration.

8. The device (100) of claim 1, wherein the burr (180) is incorporated as the most distal portion of the cutter.

9. The device (100) of claim 1, wherein the fluted cutting edge (112) is tangent to a helical path about the rotational axis of the cutter (108).

10. The device (100) of claim 1, further comprising a guidewire lumen extending through the torque shaft (114) and through the cutter (108).

11. The device (100) of claim 1, wherein a distal portion of the catheter body (120) is more flexible than a remainder of the catheter body.

12. The device (100) of claim 1, wherein the cutter (108) comprises a plurality of flutes (110) having a plurality of fluted cutting edges (112).

13. The device (100) of claim 12, wherein the fluted cutting edges (112) are helical.

14. The device (100) of claim 13, wherein each flute (110) is arranged relative to the opening (106) in the housing (104) such that during operation, a total length of the fluted cutting edge (112) exposed through the housing openings remains the same.

15. The device (100) of claim 12, wherein a portion of the housing (104) comprises a curved surface and the opening (106) forms a plane across the curved surface such that as the fluted cutting edge (112) rotates across the opening, a portion of the fluted cutting edge extends out of the housing through the opening.

## Patentansprüche

1. Vorrichtung (100) zum Entfernen von Material aus Körperlumen, wobei die Vorrichtung umfasst:
einen Katheterkörper (120) mit einem proximalen Ende, einem distalen Ende und einem sich dadurch erstreckenden Katheterlumen;
eine am distalen Ende des Katheterkörpers angeordnete Schneidanordnung (102), wobei die Schneidanordnung umfasst:
ein Gehäuse (104) mit mindestens einer Öffnung (106) ;
ein Schneidwerkzeug (108), das konfiguriert ist, um sich innerhalb des Gehäuses zu drehen, wobei das Schneidwerkzeug mindestens eine geriffelte Schneidkante (112) umfasst, die konfiguriert ist, um in einer Richtung zu schneiden, die tangential zu der Rotationsrichtung des Schneidwerkzeugs ist;
eine Torsionswelle (114), die sich durch das Katheterlumen erstreckt und ein erstes Ende hat, das mit der Schneidanordnung gekoppelt ist, und ein zweites Ende, das geeignet ist, um mit einem Drehmechanismus (150) zu koppeln, wobei eine Außenfläche der Torsionswelle eine erhabene Oberfläche umfasst, die spiralförmig darauf angeordnet und so angepasst ist, dass die erhabene Oberfläche bei Drehung Materialien in einer proximalen Richtung befördert, **gekennzeichnet durch** einen Fräser (180), der distal von der Schneidanordnung vorsteht.

2. Vorrichtung (100) nach Anspruch 1, ferner umfassend ein Rohrelement, das das Gehäuse (104) mit einem distalen Ende des Katheterkörpers (120) verbindet.

3. Vorrichtung (100) nach Anspruch 1, wobei das Schneidwerkzeug (108) zylindrisch geformt ist, ein proximaler Abschnitt des Gehäuses (104) zylindrisch ist und ein distaler Abschnitt des Gehäuses verjüngt ist.

4. Vorrichtung (100) nach Anspruch 1, wobei der Fräser (180) eine abrasive Oberfläche hat.

5. Vorrichtung (100) nach Anspruch 1, wobei der Fräser (180) stumpf ist und eine feine Körnung hat.

6. Vorrichtung (100) nach Anspruch 5, wobei die feine Körnung Diamantkörnung ist.

7. Vorrichtung (100) nach Anspruch 1, wobei der Fräser (180) spiralförmige Rillen zum Unterstützen des Ansaugens aufweist.

8. Vorrichtung (100) nach Anspruch 1, wobei der Fräser (180) als der am weitesten distal gelegene Abschnitt des Schneidwerkzeugs eingebaut ist.

9. Vorrichtung (100) nach Anspruch 1, wobei die geriffelte Schneidkante (112) einen spiralförmigen Weg um die Rotationsachse des Schneidwerkzeugs (108) tangiert.

10. Vorrichtung (100) nach Anspruch 1, ferner umfassend ein Führungsdrahtlumen, das sich durch die Torsionswelle (114) und durch das Schneidwerkzeugs (108) erstreckt.

11. Vorrichtung (100) nach Anspruch 1, wobei ein distaler Abschnitt des Katheterkörpers (120) flexibler ist als ein Rest des Katheterkörpers.

12. Vorrichtung (100) nach Anspruch 1, wobei das Schneidwerkzeugs (108) eine Vielzahl von Rillen (110) mit mehreren geriffelten Schneidkanten (112) umfasst.

13. Vorrichtung (100) nach Anspruch 12, wobei die geriffelten Schneidkanten (112) spiralförmig sind.

14. Vorrichtung (100) nach Anspruch 13, wobei jede Rille (110) relativ zu der Öffnung (106) in dem Gehäuse (104) derart angeordnet ist, dass während des Betriebs eine Gesamtlänge der geriffelten Schneidkante (112), die durch die Gehäuseöffnungen freiliegt, gleich bleibt.

15. Vorrichtung (100) nach Anspruch 12, wobei ein Abschnitt des Gehäuses (104) eine gekrümmte Oberfläche umfasst und die Öffnung (106) eine Ebene über der gekrümmten Oberfläche bildet, so dass, wenn sich die geriffelte Schneidkante (112) über die Öffnung dreht, sich ein Abschnitt der geriffelten Schneidkante durch die Öffnung aus dem Gehäuse heraus erstreckt.

## Revendications

1. Dispositif (100) pour retirer du matériau des lumens corporels, le dispositif comprenant:
un corps de cathéter (120) ayant une extrémité proximale, une extrémité distale et un lumen de cathéter s'étendant à travers celui-ci;
un ensemble de coupe (102) situé à l'extrémité distale du corps de cathéter, l'ensemble de coupe comprenant:
un logement (104) ayant au moins une ouverture (106) ;
un élément de coupe (108) configuré pour tourner à l'intérieur du logement, l'élément de coupe comprenant au moins un bord coupant cannelé (112) qui est configuré pour couper dans une direction qui est tangentielle à la direction de rotation de l'élément de coupe;
un arbre de torsion (114) s'étendant à travers le lumen du cathéter et ayant une première extrémité couplée à l'ensemble de coupe et une seconde extrémité adaptée pour être couplée à un mécanisme rotatif (150), où une surface extérieure de l'arbre de torsion comprend une surface surélevée située de manière hélicoïdale sur celui-ci et adaptée de sorte que lors de la rotation, la surface surélevée transporte les matériaux dans une direction proximale, **caractérisé par** une fraise (180) faisant saillie distale de l'ensemble de coupe.

2. Dispositif (100) selon la revendication 1, comprenant en outre un élément de tube reliant le logement (104) à une extrémité distale du corps de cathéter (120).

3. Dispositif (100) selon la revendication 1, dans lequel l'élément de coupe (108) est de forme cylindrique, une partie proximale du logement (104) est cylindrique et une partie distale du logement est conique.

4. Dispositif (100) selon la revendication 1, dans lequel la fraise (180) a une surface abrasive.

5. Dispositif (100) selon la revendication 1, dans lequel la fraise (180) est émoussée et a un grain fin.

6. Dispositif (100) selon la revendication 5, dans lequel le grain fin est un grain de diamant.

7. Dispositif (100) selon la revendication 1, dans lequel la fraise (180) a des cannelures hélicoïdales pour aider l'aspiration.

8. Dispositif (100) selon la revendication 1, dans lequel la fraise (180) est incorporée comme la partie la plus distale de l'élément de coupe.

9. Dispositif (100) selon la revendication 1, dans lequel le bord coupant cannelé (112) est tangent à une trajectoire hélicoïdale autour de l'axe de rotation de l'élément de coupe (108).

10. Dispositif (100) selon la revendication 1, comprenant en outre un lumen de fil-guide s'étendant à travers l'arbre de torsion (114) et à travers l'élément de coupe (108).

11. Dispositif (100) selon la revendication 1, dans lequel une partie distale du corps de cathéter (120) est plus flexible qu'un reste du corps de cathéter.

12. Dispositif (100) selon la revendication 1, dans lequel l'élément de coupe (108) comprend une pluralité de cannelures (110) ayant une pluralité de bords coupants cannelés (112).

13. Dispositif (100) selon la revendication 12, dans lequel les bords coupants cannelés (112) sont hélicoïdaux.

14. Dispositif (100) selon la revendication 13, dans lequel chaque cannelure (110) est agencée par rapport à l'ouverture (106) dans le logement (104) de sorte que pendant le fonctionnement, une longueur totale du bord coupant cannelé (112) exposée à travers les ouvertures du logement reste la même.

15. Dispositif (100) selon la revendication 12, dans lequel une partie du logement (104) comprend une surface incurvée et l'ouverture (106) forme un plan à travers la surface incurvée de sorte que lorsque le bord coupant cannelé (112) tourne à travers l'ouverture, une partie du bord coupant cannelé s'étend hors du logement à travers l'ouverture.
